Europäisches Patentamt

European Patent Office

Office européen des brevets

(19)

(11) **EP 0 293 443 B1**

(12) **EUROPEAN PATENT SPECIFICATION**

(45) Date of publication and mention
of the grant of the patent:
**26.02.2003 Bulletin 2003/09**

(51) Int Cl.$^7$: **C07H 21/04**

(86) International application number:
**PCT/US87/02822**

(21) Application number: **88900275.4**

(22) Date of filing: **29.10.1987**

(87) International publication number:
**WO 88/003533 (19.05.1988 Gazette 1988/11)**

(54) **CONSTRUCTION OF SYNTHETIC DNA AND ITS USE IN LARGE POLYPEPTIDE SYNTHESIS**

HERSTELLUNG SYNTHETISCHER DNS UND DEREN VERWENDUNG BEI DER SYNTHESE
GROSSER POLYPEPTIDE

STRUCTURE D'ADN SYNTHETIQUE ET SON EMPLOI DANS LA SYNTHESE DE POLYPEPTIDES
DE GRANDE DIMENSION

(84) Designated Contracting States:
**AT BE CH DE FR GB IT LI LU NL SE**

(30) Priority: **04.11.1986 US 927258**

(43) Date of publication of application:
**07.12.1988 Bulletin 1988/49**

(73) Proprietor: **PROTEIN POLYMER
TECHNOLOGIES, INC.
San Diego, CA 92121 (US)**

(72) Inventors:
• **FERRARI, Franco, A.
La Jolla, CA 92037 (US)**
• **RICHARDSON, Charles
San Diego, CA 92122 (US)**
• **CHAMBERS, James
San Diego, CA 92103 (US)**
• **CAUSEY,Stuart,C
Del Mar,CA 92014-3723 (US)**
• **POLLOCK, Thomas, J.
San Diego, CA 92122 (US)**

(74) Representative: **Harrison, David Christopher et al
MEWBURN ELLIS
York House
23 Kingsway
London WC2B 6HP (GB)**

(56) References cited:
EP-A- 0 191 748          CA-A- 1 282 020
GB-A- 2 162 190          JP-A- 61 085 400
US-A- 4 585 585          US-A- 4 589 882

• **S. TABOR et al. "A Bacteriophage T7 RNA
Polymerase/Promoter System for Controlled
Exclusive Expression of Specific Genes",
Proceedings of the National Academy of
Sciences USA, Volume 82, pages 1074-1078,
published February 1985 by the National
Academy of Sciences of the United States of
America (Washington, D.C., USA). see Entire
Document.**
• **F.W. STUDIER et al, "Use of Bacteriophage T7
RNA Polymerase to Direct Selective High-Level
Expression of Cloned Genes", Journal of
Molecular Biology, Volume 189, Number 1,
pages 113-130, published 5 May 1986 by
Academic Press (New York, NY, USA). see Entire
Document.**
• **A. ROSENBLUH et al., "Identification of a New
Developmental Locus in Bacillus Subtilis by
Construction of a Deletion Mutation in a Cloned
Gene under Sporulation Control", Journal of
Bacteriology, Volume 148, Number 1, pages
341-351, published October 1981 by the
American Society for Microbiology
(Washington, D.C., USA). see Abstract.**
• **GENE vol. 39, 1985, pages 239 - 245**
• **CHEMICAL ABSTRACTS, vol. 93, 1989,
Columbus, Ohio, US; abstract no. 93:95634S**
• **CHEMICAL ABSTRACTS, vol. 88, no. 294, 1978,
Columbus, Ohio, US; abstract no. 166571D,**
• **J.BIOL.CHEM. vol. 253, 1978, page 2044**
• **EXPERIENTIA vol. 41, 1985, pages 1167 - 1171**
• **YONEHARA H.: 'PEPTIDE CHEMISTRY', 1979 *
page 169 - page 174 ***
• **BIOTECHNOLOGY vol. 3, 1985, page 671**
• **J.PROTEIN CHEM vol. 3, 1984, pages 403 - 436**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

- **SCIENCE vol. 225, 1984, pages 593 - 599**
- **BIOTECHNOLOGY September 1983, pages 602 - 609**
- **PATHOL.BIOL. vol. 33, 1985, pages 266 - 274**
- **NUCLEIC. ACIDS. RES. vol. 12, 1984, page 6797**
- **GENE ANAL TECHN. vol. 2, 1985, pages 77 - 82**

**Description**

INTRODUCTION

Technical Field

The field is related to the production of high-molecular-weight polymers, either nucleic acids or peptides that are the expression products of the nucleic acids, and is particularly related to the production of high-molecular-weight peptides containing repeating sequences by biochemical processes, the peptide finding use as structured materials.

Background

Recombinant DNA technology has been applied in the isolation of natural genes and the expression of these genes in a variety of host cells. Typically, this technology has had utility in producing biologically active polypeptides. such as interferons or peptide hormones, which were impractical to produce in useful amounts by other means. It was also possible to produce modified proteins by isolating natural genes and utilizing the techniques of site specific, in vitro mutagenesis to alter these genes and thereby change the polypeptides produced. Other polypeptides have been created by combining sections of various native genes to produce new polypeptides that are chimeric molecules of the several naturally occurring molecules.

With the advent of efficient and automated methods for the chemical synthesis of DNA, it has become possible to synthesize entire genes and to modify such synthetic genes at will during the course of synthesis. However, these various technologies have been applied to the production of natural or modified versions of natural polypeptides. There have been very few attempts to use these technologies to create substantially new polypeptides. In nature, polypeptides have a wide range of chemical, physical and physiological characteristics. Nevertheless there are commercial applications for which known, naturally occurring polypeptides are not appropriate.

While biotechnology is versatile, usually it has been limited in its applications to naturally occurring products or modifications of naturally occurring molecules. One great strength of organic chemical synthesis, by contrast, has been the ability to transform inexpensive carbon materials to a wide variety of polymeric molecules, including naturally occurring molecules, but most importantly entirely new chemical structures, such as polypropylene and polyacrylates, which have defined and predicted chemical properties not associated with naturally occurring molecules.

Such materials, particularly high-molecular-weight polymers containing repeating sequences of amino acids, have proven difficult to produce by biochemical means. The genes necessary for producing large peptides containing repeating units of amino acids were unstable and often underwent intermolecular recombination causing deletions of repeating units in the gene. The development of a biotechnology which would produce polymeric molecules by biological processes similar to those available by organic synthesis would significantly broaden the range of applications of biotechnology.

Brief Description of the Relevant Literature

The cloning of multiple lactose operators up to four in tandem is disclosed by Sadler et al., Gene, (1980) 8: 279-300. Hybrid bacterial plasmids containing highly repeated satellite DNA is disclosed by Brutlag et al., Cell, (1977) 10:509-519. The synthesis of a poly(aspartyl-phenylalanine) in bacteria is disclosed by Doel et al., Nucleic Acids Research, (1980) 8:4575-4592. A method for enriching for proline content by cloning a plasmid which codes for the production of a proline polymer was disclosed by Kangas et al., Applied and Environmental Microbiology, (1982) 43: 629-635. The biological limitations on the length of highly repetitive DNA sequences that may be stably maintained within plasmid replicons is discussed by Gupta et al. in Bio/Technology, p. 602-609, September 1983.

GB-A-2162190 provides a paper proposal of the possibility of producing silk proteins using recombinant microorganisms or in tissue culture. The possibilities of producing materials identical to silk proteins and materials which are modified are also disclosed.

SUMMARY OF THE INVENTION

Methods are provided according to the appended claims for the production of polypeptides having repetitive oligomeric units by expression of a synthetic structural gene. The individual units coding for an oligomeric peptide sequence are varied as to nucleotide sequence utilizing amino acid codon redundancy. Long nucleic acid sequences are built up by synthesizing nucleic acid oligomers which express a plurality of individual repetitive peptide units, and the oligomers are joined to provide a polynucleotide of the desired length. Expression systems are used which provide for the growth of the subject host to high density prior to significant expression of the polypeptide product, followed by induction of expression to provide high yields of the polypeptide product, which can be isolated from the host cells. In one embodiment, a system is employed where the transcription initiation system of the synthetic gene is not recognized by the host RNA polymerase and a gene expressing a functional RNA polymerase under inducible regulation is included in the host.

BRIEF DESCRIPTION OF THE FIGURES

Figure 1: Plasmid pSY701 structure.
Figure 2: Immunoblots of polypeptide products using antibody to (A) beta-lactamase or to (B) gly-ala peptide.
Figure 3: Construction flowchart for plasmid pG10/SlpI.
Figure 4: Immunoblots of polypeptide products (A) T7gp10/SlpI with anti-Slp Ab, (B) T7gp9/SlpI with anti-Slp Ab, or (C) staining with Coomassie blue.

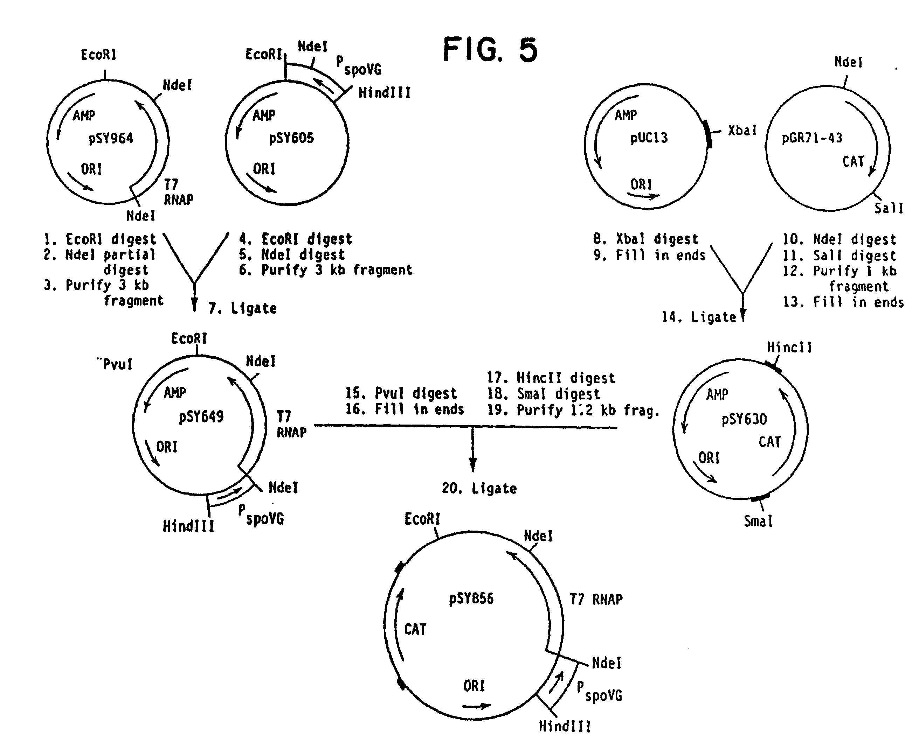

Figure 5: Construction flowchart for plasmid pSY856.

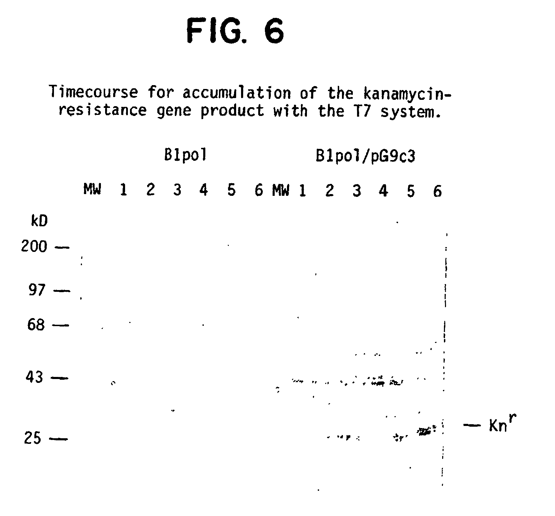

Figure 6: Time course for accumulation of the kanamycin-resistance gene product with the T7 system.
Figure 7: Construction flowchart for plasmid pSY857.
Figure 8: Construction flowchart for plasmid pSY980.
Figure 9: (A) Amido black stain of gel containing the product of beta-galactosidase/SlpIII gene fusion; (B) immunoblot of same product with anti-Slp antibody.

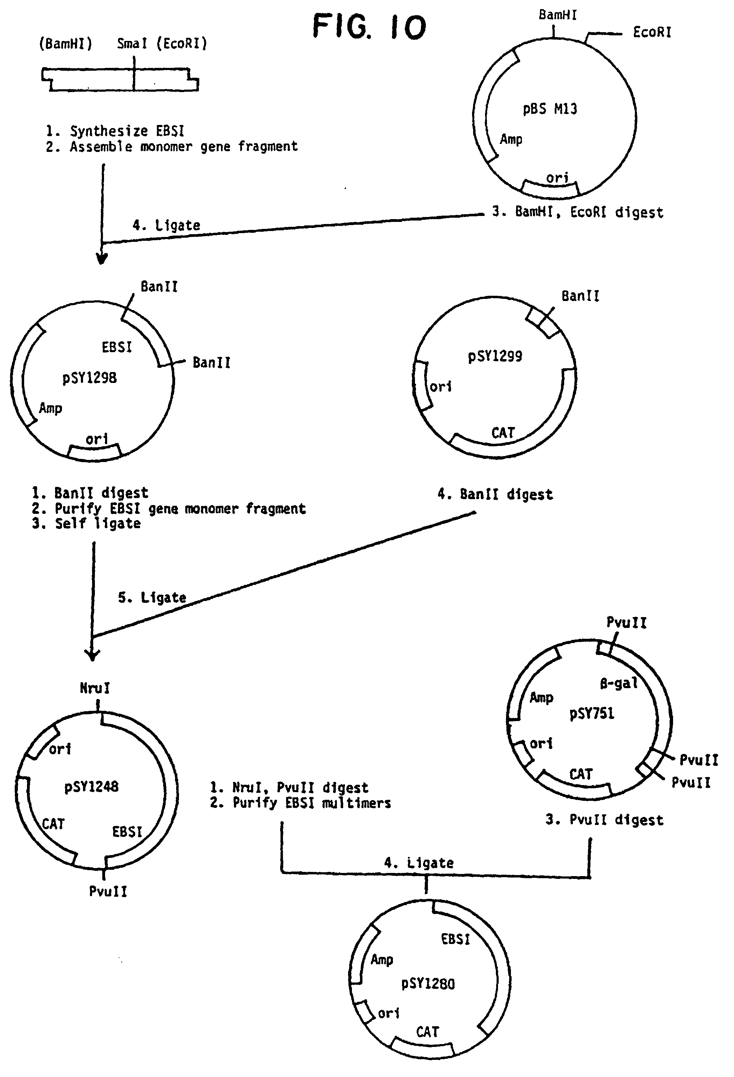

Figure 10: Construction flowchart for plasmid pSY1280.

DESCRIPTION OF PREFERRED EMBODIMENTS

DNA sequences to which this invention relates encode polypeptides which are polyoligomers of repeating, relatively short, amino acid sequence units. The oligomers may be linked by spacers of different amino acid sequence. The polypeptides therefore contain repetitive amino acid sequences and are particularly useful as fibrous proteins, including elastomeric. The gene encoding the repeating-unit-containing peptides is produced to particularly avoid problems previously associated with genes containing multiple repeating units.

The genes produced according to the subject invention comprise multimers of DNA sequences encoding the same amino acid sequence unit, where two or more different multimers encoding different amino acid units may be joined together to form a block copolymer. The individual units will have from 4 to 30 amino acids (12 to 120 nt), more usually 4 to 25 amino acids (12 to 75 nt), particularly 4 to 8 amino acids, usually having the same amino acid appear at least twice in the same unit, generally separated by at least one amino acid. The units of the multimer coding for the same amino acid sequence involve two or more nucleotide sequences, relying on the codon redundancy to achieve the same amino acid sequence.

Subject to the limitation of the appended claims, for the most part the DNA compositions produced according to this invention may be depicted by the following formula:

$$K_k (W M X_x N Y_y)_i L_l$$

wherein:

K is a DNA sequence encoding an amino acid sequence of from about 1 to 100 amino acids, usually 1 to 60 amino acids, which may be any sequence, generally being fewer than about 20% of the total number of amino acids, more generally being fewer than about 10% of the total number of amino acids, which may be any sequence, particularly a naturally occurring sequence where the multimer structural gene has been fused to another DNA sequence in reading frame. K will have the initiation methionine codon.

k is 0 or 1;
W has the formula:

$$[(A)_n (B)_p]_q$$

wherein:

A is a DNA sequence coding each time that it appears for the same amino acid sequence unit normally having at least one amino acid appear at least twice in the sequence, where A will generally be from about 12 to 90 nucleotides (nt), more usually for about 12 to 75 nucleotides;

where there will usually be at least two different A's, usually not more than ten different A's, more usually not more than six different A's, which code for the same amino acid sequence but differ from each other by at least one nucleotide and may differ by as many as ten nucleotides, usually not differing by more than about five nucleotides from another A sequence, each of the different A's usually being repeated at least twice; at least two different codons are employed for the same amino acid, e.g., GGC and GGA for glycine, in different A's coding for the same amino acid sequence unit;

n will be an integer of at least 2, usually at least about 8, and not more than about 250, usually not more than about 200, frequently not more than about 125, and in some instances may not exceed about 50;

B is a DNA sequence different from A coding for an amino acid sequence other than the amino acid sequence unit coded by the A unit and serves as a linking unit between oligomers of A units. B will generally have from about 3 to 45 nt, (1 to 15 amino acids) more usually from about 3 to 30 nt (1 to 10 amino acids);

where the B units appearing in the gene may be the same or different, there usually not being more than about 10 different B units, more usually not more than about 5 different B units, where the B units may differ in from 1 to 45 nt, more usually from about 1 to 15 nt, where the different B's may code for the same or different amino acid sequence;

p is 0 or 1 and may differ each time there is a successive A unit;

q is an integer of at least 1 and will vary with the number of nucleotides in A and B, as well as the values of n and p. The variable q will be selected so as to provide for at least 90 nucleotides for the multimeric portion of the structural gene, preferably at least about 150nt, more preferably at least 450nt, and most preferably at least 900 nucleotides, and the number of nucleotides will usually not exceed about 10,000, more usually not exceeding about 8,000, generally being in the range of about 900 to 6,000, more usually to about 5,000; and

M is a DNA nucleotide sequence of 0 to 18 nt, which may encode any amino acid sequence, usually including amino acids of A and/or B, generally limited to the amino acids of A and/or B;

X may be the same as or different from W, usually different, and will have the formula

$$[A^1)_{n^1} (B^1)_{p^1}]_{q^1}$$

wherein:

$A^1$, $B^1$, $N^1$, $p^1$ and $q^1$ are the same as or different from A, B, n, p and q respectively, at least one being different, wherein the analogous symbols come within the same definition as their counterparts;

x is 0 or 1 ;

N is the same as or different from M and comes within the same definition as M;

Y may be the same as or different from W, usually different, and will have the formula

$$[A^2)_{n^2} (B^2)_{p^2}]_{q^2}$$

wherein:

$A^2$, $B^2$, $n^2$, $p^2$ and $q^2$ are the same as or different from A, B, n, p and q respectively, at least one being different, wherein the analogous symbols come within the same definitions as their counterparts.

y is 0 or 1 ;

i is 1 to 100, usually 1 to 50, more usually 1 to 30, particularly 1, when x and y are 0;

when x or y are 1, q, $q^1$ and $q^2$ will be a total of at least 2, usually at least 5 and not more than about 50, usually not more than about 30.

The total number of nucleotides will preferably be at least abut 900nt and may be 20knt (kilonucleotides), usually not more than about 15knt, more usually not more than about 10knt.

The polypeptide encoded by the above DNA sequence will have the fallowing formula:

$$K'_k (W' \: M' \: X'_x \: N' \: Y'_y)_i \: L'_l$$

wherein:

W' will have the following formula

$$[(D)_n\ (E)_p]_q$$

wherein:

D is the amino acid sequence encoded for by A and therefore has the numerical limitations based on 3 nucleotides defining a codon that codes for one amino acid;

E is the amino acid sequence encoded for by B, and therefore has the numerical limitations based on 3 nucleotides defining a codon, where each E may be the same or different, depending upon the coding of B;

and, wherein, likewise K', W', M', X', N', Y' and L' is the amino acid sequence encoded for by K, W, M, X, N, Y and L respectively. However, in the case of K and L, subsequent processing, such as protease treatment, cyanogen bromide treatment, etc. may result in partial or complete removal of the N- or C-terminal non-multimeric chains.

n, p, q, k, i and l have the same definitions as previously indicated.

Particular polymeric compositions having repeating multimeric units having the same compositions (A) will have the following formula where x and y are 0,

$$K'_k\ [(D)_n\ (E)_p]_q\ L'_l$$

where all of the symbols have been defined previously; and

the DNA sequence will have the formula

$$K_k\ [(A)_n\ (B)_p]_q\ L_l$$

where all of the symbols have been defined previously.

Particular DNA sequences encoding copolymeric compositions having a repeating unit of two to three multimeric blocks will have the following formula:

$$K_k\ (W''\ M''\ X''\ N''\ Y''_{y''})_{i''}\ L_1$$

wherein:

W'' is a multimer having the formula

$$[(A^3)_{n^3}\ (B^3)_{p^3}]_{q^3},$$

where $A^3$ is of 4 to 8, usually 4 to 6 codons, otherwise coming within the definition of A;

$n^3$ will be from about 2 to 12, usually 2 to 10;

$B^3$ is of from 2 to 8, usually 4 to 6 codons;

$p^3$ is 0 or 1 ;

$q^3$ is of from about 2 to 25, usually 2 to 20;

X'' and Y'' are the same as or different from W'', usually different, coming within the same definitions as W'';

M'' and N'' come within the definitions of M' and N';

i'' is at least 2, usually at least 5 and not more than about 75, usually not more than about 50, generally not exceeding 30;

with the other symbols as defined previously.

The polypeptide compositions encoded by DNA produced according to the invention will have a molecular weight of at least about 10kDal, preferably 15kDal and may have molecular weights as high or higher as 400kDal, usually not exceeding 300kDal, more usually not exceeding about 250kDal, the higher ranges generally being the multimer combinations, with the individual multimer usually being less than about 150kDal, usually less than about 100 kDal.

The nucleotide sequences which are employed will be synthesized, so that the repetitive units will have different codons for the same amino acid as described above. Usually, at least about 25%, more usually at least about 40%, and generally at least about 60%, but not greater than about 95%, preferably not greater than about 90% of the nucleotide sequences encoding the repetitive units will be the same. Greater diversity within those ranges will be employed where the initial constructs are experimentally shown to undergo spontaneous recombination events.

Of particular interest are polypeptides which have as a repeating unit SGAGAG (G = glycine; A - alanine; S - serine). This repeating unit is found in a naturally occuring silk fibroin protein, which can be represented as GAGAG (SGAGAG)$_8$SGAAGY (Y = tyrosine). In the subject invention, the repeating unit is designed where the N-terminus may be MGAGAG or any other sequence of generally at least about 3 amino acids, usually at least about 5 amino acids, more usually 12 amino acids and not greater than 200, usually not greater than 100 amino acids, which may be different from the repetitive unit. Generally, a different N-terminus will be the result of insertion of the gene into a vector in a manner that results in expression of a fusion protein. Any protein which does not interfere with the desired properties of the product may provide the N-terminus. Particularly, endogenous host proteins, e.g. bacterial proteins, may be employed. The choice of protein may depend on the nature of the transcriptional initiation region. Similarly, the C-terminus may have an amino acid sequence different from the repeat sequence. Conveniently, there may be from 1 to 100, usually 1 to 25 amino acids, which may be the C-terminus of a naturally occurring structural gene, which again typically results from the formation of a fusion product.

A silk-like-protein (Slp) gene may be produced by providing oligomers of from about 5 to 25 repeat units as described above, more usually of about 10 to 20 repeat units. By having different cohesive ends, the oligomers may be concatemerized to provide for the polymer having 2 or more of the oligomeric units, usually not more than about 50 oligomeric units, more usually not more than about 30 oligomeric units, and frequently not more than about 25 oligomeric units.

The silk-like proteins may be varied by having alternate multimers with the same or different handedness. For example, in the formula, (B)$_p$ may provide an even or odd number of amino acids. In silk, the hydrogens of the glycine may align on one side and the methyls and hydroxyls of alanine and serine on the other. If (B)$_p$ is even, there will be continuous alignment, if odd, there will be alternating alignment of (A)$_n$. Thus, different properties can be achieved by changing the number of amino acids encoded by (B)$_p$.

Of particular interest are polypeptides which mimic the composition and physical properties of silk of <u>Bombyx mori</u>.

Also of interest are polypeptides which have as a base repeating unit GVGVP (G = glycine, V = valine, P = proline), which may be found in naturally occurring elastin. In the subject invention, the N-terminus may be any convenient sequence and, if desired, may be in whole or in part removed by a protease. Usually the N-terminal sequence which does not have the subject motif will be less than about 100 amino acids, more usually less than about 60 amino acids.

Of particular interest is a base sequence of about 6 to 10, preferably 8, units separated by a sequence of about 6 to 20 amino acids, usually 8 to 16 amino acids, which may include an internal repeat different from the basic repeating unit of from 4 to 8 amino acids. For example, the second repeat sequence could be GAGAGS, repeated twice. The total number of base repeating units will generally be in the range of about 150 to 300, usually 175 to 250. The C-terminus may terminate with a repetitive unit or portion thereof or a different sequence of from 1 to 100, usually 1 to 30 amino acids. The C-terminus is not critical to the invention and will be selected primarily for convenience. As with the N-terminus, it may be designed for proteolytic cleavage. As in the case of the silk protein, the subject elastin-like protein may be similarly engineered.

Of particular interest are proteins which mimic the properties of elastin and provide for elastomeric properties.

The copolymer involving repeating units is a powerful method for varying properties, by appropriate choice of the different units, the number of units in each multimer, the spacing between them, and the number of repeats of the multimer combination assembly. Thus, by varying the number and arrangement of primary monomers, a variety of different physical and chemical properties can be achieved.

Exemplary of the use of the block copolymers are combinations of silk units and elastin units to provide products having properties distinctive from polymers only having the same monomeric unit.

To prepare the structural genes, various approaches can be employed. To prepare the oligomers, complementary strands of DNA may be synthesized, so that upon hybridization double-stranded DNA is obtained with the appropriate termini. If desired, each of the oligomeric units may be the same, so that in a single step, a concatemer may be obtained depending upon the conditions of the hybridization. Normally, conventional annealing and ligating conditions will be employed, such as are described in the examples that follow.

If desired, two different oligomeric units may be prepared where the termini of the two units are complementary one with the other but the termini of the same unit are unable to bind together. In this way one can build individual oligomeric units and then join them together to form the concatemer, where the intervening linking sequences are defined at least in part by the termini. Depending upon the construct, the 5' terminus may provide for the initiation codon methionine, or the structural gene may be joined to an adapter which may provide for a unique sequence (optionally cleavable by a specific enzyme) at the 5' terminus or may be inserted into a portion of gene, usually endogenous to the host, in proper reading frame so as to provide for a fusion product. By providing for appropriate complementary termini between the adapter or truncated gene and the 5' end of the subject structural gene, the sequences can be joined in proper reading frame to provide for the desired protein. Advantages that may be achieved by employing adapters or fusion proteins include having specific sequences for special purposes, such as linking, secretion, complex formation with other proteins, affinity purification, or the like.

Once the structural gene has been assembled, it may be cloned; clones having the desired gene, particularly as to sequence length, may be isolated; and the gene may be removed and used to join to a sequence for expression.

The expression construct will include transcriptional and translational initiation and termination regulatory regions, 5' and 3', respectively, of the structural gene. As already indicated, these regions may be created by employing a fusion protein, where the subject structural gene is inserted into a different structural gene downstream from its initiation codon and in reading frame with the initiation codon. Alternatively, various transcriptional and translational intiation regions are available from a wide variety of genes for use in expression hosts, so that these transcriptional and translational initiation regions may be joined to the subject structural gene to provide for transcription and translation initiation of the subject structural genes. A wide variety of termination regions are available which may be from the same gene as the transcriptional initiation region or from a different gene. Numerous constructs have been disclosed in the literature, and the same procedures may be applied with the subject gene as have been employed with other structural genes.

Of particular interest is the use of an inducible transcription initiation region. In this manner, the host strain may be grown to high density prior to significant expression of the desired product. Providing for inducible transcription is particularly useful where the peptide is retained in the cellular host rather than secreted by the host.

A number of inducible transciption initiation regions exist or can be employed in particular situations. The inducible regions may be controlled by a particular chemical, such as isopropyl thiogalactoside (IPTG) for inducing the beta-galactosidase gene. Other inducible regions include lambda left and right promoters; various amino acid polycistrons, e.g., histidine and tryptophan; temperature sensitive promoters; and regulatory genes, e.g., cI$^{ts}$ 857.

An alternative system which may be employed with advantage is use of a combination of transcription initiation regions. A first transcription initiation region which regulates the expression of the desired gene but which is not functional in the expression host by failing to be functional with the endogenous RNA polymerase is employed. A second transcription initiation region, such as an inducible region, can then be employed to regulate the expression of an RNA polymerase with which the first transcription initiation region is functional. In this manner expression only occurs upon activation of the regulatory region controlling the expression of the exogenous RNA polymerase. In the subject application, this system is illustrated with the T7 phage transcription initiation region, specifically the initiation regions of genes 9 and 10 of T7 phage.

An alternative system relies on the use of mutants which undergo a developmental change based on a change in the environment, such as a lack of a nutrient, temperature, osmotic pressure, salinity, or the like. Illustrative of this system, strains of B. subtilis can be obtained which are incapable of sporulation but which can produce those components which initiate expression of products involved with sporulation. Therefore, by a change in the condition of the medium, a transcription initiation region associated with sporulation will be activated. In this situation, the host provides the necessary inducing agent or activator to initiate expression.

Various other techniques exist for providing for inducible regulation of transcription and translation of a gene in a particular host.

For the most part, the host will be a unicellular organism, either a prokaryote or a eukaryote, selected from bacteria, algae, fungi, filamentous fungi, etc. Illustrative hosts include E. coli, B. subtilis, B. stearothermophilus, S. cerevisiae, and the like.

The expression construct for expression of the desired gene, by itself or in conjunction with any auxiliary genes involved with transcription, will normally be joined to an appropriate vector for introduction into the expression host. A large number of vectors are commercially available with others being described in the literature. The vectors are normally characterized by having one or more unique restriction sites, a replication system for extrachromosomal maintenance in the host, and one or more markers which allow for selective pressure on the host. The markers may provide complementation, prototrophy to an auxotrophic host, resistance to a biocide, e.g., an antibiotic such as penicillin or kanamycin, or the like. In some instances, rather than selective pressure, a marker gene is employed which allows for detection of particular colonies containing the gene. This situation is illustrated by the gene for beta-galactosidase, where a substrate is employed which provides for a colored product.

The expression construct, including any auxiliary genes, may be introduced into the expression vector in accordance with known techniques, particularly employing restriction, insertion, and ligation.

The expression construct may then be used for transformation of the appropriate host. Depending upon the host, either intact cells or protoplast may be employed, where transformation or conjugation is employed. Conveniently, calcium-phosphate-precipitated DNA or non-ionic detergents may be employed to introduce the plasmid into the host. It should be appreciated that it is not necessary to employ vectors for host transformation, since bare DNA can be introduced for integration into the genome. However, even where integration is desired, a much greater efficiency of integration is achieved employing the vectors, thus favoring the employment of vectors.

Depending upon the nature of the vector, the expression construct may be maintained on an extrachromosomal element or become integrated into the host. Where integration is desired, it will usually be desirable with prokaryotes and some eukaryotes to have a sequence homologous to a sequence in the chromosome of the host. Usually the sequence will be at least about 200 bp and not more than about 5000 bp, usually not more than about 2000 bp. The choice of the homologous sequence is somewhat arbitrary, but may be used for complementation, where the host is an auxotrophic mutant and the homology provides prototrophy.

The transformants or integrants may then be grown in an appropriate nutrient medium to high density, followed by induction of transcription in accordance with the nature of the transcriptional system of the expression construct. Where the desired protein is retained in the cytoplasm, these cells are harvested and lysed, and, depending upon the use of the protein, the protein may be further purified in accordance with conventional techniques, such as chromatography, solvent-solvent extraction, affinity chromatography, and the like.

The repetitive proteins can find a variety of uses. The Slp proteins may be used in producing fibers having unique properties, as a substitute for silk, and the like. Collagen proteins can be produced, where the collagen is free of the telopeptide or contains the telopeptide, depending upon its function. Atelopeptidecollagen should have little if any immunogenicity, so as to be a useful structural element for a variety of prosthetic devices or for use as a collagen substitute in other applications. Similarly, other proteins having repetitive sequences, such as keratin, can also be prepared in accordance with the subject invention. Other useful repetitive proteins can be prepared based on sequences of spider silks and other repetitive animal fibers. Artificial peptides useful for immunization could also be prepared based on repeating sequences present in various surface antigens of disease-causing microorganisms, such as parasites, bacteria, and viruses.

The following examples are offered by of illustration and not with limitation.

Example 1

DNA Preparation Methods

1.    Preparation of plasmid DNA from E. coli:

A. Small scale: Plasmid DNA was prepared from 1.5 ml cultures by either the boiling procedure or the alkaline lysis method (Maniatis, et al., Molecular Cloning: A Laboratory Manual. Cold Spring Harbor Laboratory, Cold Spring Harbor. (1982)).
B. Large scale: A plasmid-carrying strain was grown overnight in 1 liter of Luria broth with the appropriate antibiotic. The cells were collected by centrifugation at 10,000xg for 5 min and resuspended in 10 ml of ice cold TE (10mM Tris-HCl pH 8, 1mM EDTA). The cells were centrifuged again, resuspended in 4 ml of TES (TE and 25% w/v sucrose) and homogenized by vortexing. The samples were kept on ice for the following steps. Lysozyme (1 ml of 10 mg/ml) was added to the cell suspension and incubated for 5 min before the addition of 2 ml of 0.5M EDTA pH 8. After 10 min incubation, 50 ml of proteinase K (40 mg/ml) were added followed 10 min later with 15 ml of lysing buffer (0.1% Triton X-100, 1mM EDTA, 50mM tris-HCl pH 8). After 15-20 min, the cell lysate was centrifuged at 35,000xg for 90-120 min. The supernatant (19.8 ml) was transferred to a plastic tube with 20 g of CsCl and 400 $\mu$l of ethidium bromide (10 mg/ml). After dissolution, the mixture was divided into two polyallomer ultracentrifuge tubes, sealed with heat and centrifuged in a Beckman Ti 65 motor at 60,000 rpm for 24 hr. The lower plasmid DNA band was removed from the tube with a hypodermic needle. The ethidium bromide was extracted three times with an equal volume of NaCl-saturated isopropanol. Two volumes of $H_2O$ were added to the DNA solution, and then the DNA was precipitated with ethanol.

2.    Preparation of double-stranded DNA:

A culture of JM103 was grown to an $OD_{600}$ of about 0.2 and then divided into aliquots of 2 ml. Each aliquot was infected with a fresh plaque of M13 and incubated at 37°C for about 6 hr with vigorous shaking. Then the cells were pelleted and the supernatant was saved for subsequent infections. The double-stranded phage DNA was extracted by the boiling method (Maniatis et al.).

### 3. Deproteinization:

Phenol extraction was performed on a convenient volume of DNA sample, typically between 100 µl to 10 ml. The DNA sample was diluted in 0.01M Tris-HCl pH 7.5, 1mM EDTA and an equal volume of water-saturated phenol was added. The sample was vortexed briefly and placed on ice for 3 min. After centrifugation for 3 min in a microfuge, the aqueous layer was removed to a new tube and extracted once with an equal volume of chloroform:isoamylalcohol (24:1).

### 4. Ethanol precipitation:

DNA in an aqueous buffer was concentrated by ethanol precipitation. To the DNA sample was added 1/10 volume of 3M sodium acetate pH 7.5 and 2-3 volumes of cold ethanol. The DNA was precipitated for 30 min at -70°C or overnight at -20°C and then pelleted by centrifugation in the microfuge for 15 min at 4°C. The pellet was washed once with 200 µl of cold 80% ethanol and pelleted again for 10 min at 4°C. After air drying or lyophilization, the pellets were resuspended in the appropriate buffer.

### 5. Phosphatase treatment of DNA:

Phosphatase treatment of DNA was performed by adding 1 µl (25 units) of calf intestinal phosphatase (Boeringer Mannheim) directly to the restriction enzyme digestion reaction and continuing the incubation for 30 min at 37°C. The phosphatase was inactivated for 60 min at 65°C prior to deproteinization by phenol extraction.

### 6. Fill-in reaction with DNA polymerase I:

DNA was resuspended in buffer containing 50mM Tris-HCl pH 7.4, 50mM KCl, 5mM $MgCl_2$, and 400 µM each of the four deoxynucleotide triphosphates. Ten units of Klenow DNA polymerase (BRL) were added, and the reaction was allowed to proceed for 15 min at room temperature. The DNA was then phenol extracted and ethanol precipitated.

### 7. T4 polynucleotide kinase reaction:

The reaction (10 µl) contained: T4 polynucleotide kinase (BRL), 150 ng of DNA, 1 µl of 10x kinase buffer (0.7M Tris-HCl pH 7.6, 0.1M $MgCl_2$, 50mM DTT) and [$^{32}$P]-ATP (200-300 nCi). This was incubated at 37°C for 30 min and then the DNA was purified using a NACS column (Bethesda Research Labs).

### 8. Digestion with restriction endonucleases:

DNA was digested with restriction endonucleases (REM) in 1 x "AA" buffer [10 x AA buffer is 330 mM Tris-acetate, pH 7.9, 660mM potassium acetate, 100mM magnesium acetate, 50mM dithiothreitol (DTT) and 1 mg/ml bovine serum albumin (nuclease free)]. Whenever possible, the concentration of DNA was kept below 1 µg/25 µl. Incubation was at 37°C for 1-4 hrs for most restriction endonucleases except for BalI, BanI and NaeI digestions which were incubated overnight.

### 9. Analytical agarose gel electrophoresis of DNA:

To DNA samples for gel analysis we added 0.2 volumes of loading buffer (5 x electrophoresis buffer, 0.01% bromphenol blue dye, 50mM EDTA, and 50% glycerol). Then the samples were loaded into lanes of a horizontal submerged electrophoresis unit containing a 1.0% (w/v) agarose gel. The electrophoresis buffer was either 1 x TAC or 1/2 x TBE. The 1 x TAC is 40mM Tris-base, 10nM EDTA, adjusted to pH 7.8 with acetic acid. The 1/2 x TBE is 0.045M Tris-base, 0.045M boric acid, 1mM EDTA, pH 8. The gel was run at 40-50V for 18 hr, then removed and stained with 0.5 µg/ml ethidium bromide for 30 min. The DNA bands were visualized on a long wavelength UV transilluminator.

### 10. Preparative agarose gel electrophoresis:

The procedures and materials are the same as for the analytical agarose gel electrophoresis. The only difference is the use of low melting point agarose, ranging in concentration from 0.5 to 2.5% (w/v) depending on the size of the DNA fragment to be purified. DNA restriction fragments were excised from the LMP agarose gels after visualization with ethidium bromide.

11. NACS purification:

Gel fragments containing DNA were melted at 70°C for 5 min and diluted approximately 5 fold with TE1 (10mM Tris-HCl pH 7.5, 0.2M NaCl). The gel solution was applied to a MACS column (BRL). The column was washed with 5 ml of the same buffer. The bound DNA was eluted with 300 μl of either TE2 (10mM Tris-HCl pH 7.5, 1.0M NaCl for DNA fragments smaller than 1000 bp or TE3 (10mM Tris-HCl pH 7.5, 2M NaCl) for larger fragments. The eluted DNA was concentrated by ethanol precipitation.

12. DNA ligation:

Reactions for ligating cohesive ends contained: 1 μg DNA, 1 x AA buffer (see step 8, above) 1mM ATP and 20 units of T4 DNA ligase (BRL) in a 20 μl final reaction volume. The ligation was allowed to proceed for 16-18 hr at 15°C or 1-2 hr at room temperature. For blunt-ended ligations the reactions contained 1 μg DNA, 25mM Tris-HCl pH 7.5, 5mM $MgCl_2$, 5mM DTT, 0.25mM spermidine, 200mg BSA, 1mM hexamine cobalt chloride (HCC), 0.5mM ATP and 400 units T4 DNA ligase (NEB) in a 20 μl reaction volume. The ligation was allowed to proceed for 30 min to 1 hr at room temperature.

Bacterial Transformation Methods

1. Preparation of transformation-competent E. coli cells:

A culture of 200 ml of sterile L broth was inoculated with a small loopful of E. coli cells. This was incubated with shaking at 37°C until the $OD_{600}$ was approximately 0.5. The culture was placed on ice for 10 min and centrifuged at 6,000xg for 10 min. The cell pellet was resuspended in 100 ml of ice-cold 0.1M $MgCl_2$, kept on ice for 30-40 min and centrifuged again. The pellet was resuspended in 2 ml of ice-cold 100mM $CaCl_2$, transferred to a sterile test tube and incubated on ice for 24 hr. The competent cells were then aliquoted and stored at -70°C.

2. Transformation of E. coli:

An aliquot of frozen competent cells were thawed on ice. To 50 μl of cells 0.1 to 1 μg of DNA was added and the mixture was incubated on ice for 30 min. The tube was removed from ice and placed in a 42°C bath for 2 min. L broth (1 ml) was added and the transformation mix incubated with shaking at the desired temperature (usually 30°C or 37°C) for 2 hr. Then one-tenth of the transformation was plated on L broth plates containing the appropriate antibiotic and, when necessary, XGAL and IPTG were added.

3. DNA transformation of B. subtilis:

B. subtilis cells were grown to early stationary phase (change in Klett units of ≤5% in 15 min.). Transformation followed established procedures (Anagnostopoulos et al., 1981) (ref. 8). Cells (0.45 ml) were incubated with 1-10 μg of DNA at 37°C for 80 min with shaking, and then plated on TBAB agar plates with an appropriate antibiotic.

4. Isolation of plasmid DNA from B. subtilis:

Plasmid DNA from B. subtilis was obtained by a method similar to the alkaline-lysis method except that pelleted cells were resuspended in 8 ml of solution 1 (50mM glucose, 10mM EDTA, 25mM Tris-HCl (pH 8.0), 10 mg/ml lysozyme) and incubated at room temperature for 30 min. Then 16 ml of solution 2 (0.2N NaOH, 1% (w/v) SDS) was added and incubated on ice for 10 min. Finally, 12 ml of 3M potassium acetate (pH 4.8) was added and incubated an additional 20 min on ice. The lysed cells were centrifuged 15 min at 15,000 rpm in a Sorval SS-34 rotor. The DNA was precipitated by adding an equal volume of isopropyl alcohol and centrifuged at 7,000 rpm. The pellet was resuspended in 5 ml of 10mM Tris-HCl (pH 7.5), 1mM EDTA (TE). The solution was phenol extracted once and chloroform extracted. DNA was precipitated with ethanol and resuspended in 3 ml of TE. The volume was adjusted to 5.2 ml by adding 4.2g CsCl, 400 μl of ethidium bromide at 10 mg/ml and TE. The solution was transferred to a Beckman quick-seal polyallomer centrifuge tube and centrifuged at 45,000 rpm in a Beckman vti65 rotor for 18 hr.

Antibody Production, Protein Chemistry and Electrophoresis of Proteins

1.    Preparation of antibody to artificially synthesized peptides:

Synthetic peptide of sequence (GAGAGS)$_8$GAAGY was coupled to BSA using the gluteraldehyde procedure of Kagen and Glick (1979). The degree of coupling was monitored using trace amounts of radioactive iodinated synthetic peptide. Peptide conjugates at a concentration of 1mg/ml in complete Freund's adjuvent were used to immunize rabbits at day 0. Animals were re-injected with antigen in Freund's incomplete adjuvant at day 30 and titered at day 60. Positive sera was detected using a microtiter RIA using the synthetic peptide as antigen. Kagen and Glick (1979), in Methods of Radioimmunoassay, Jaffe and Berman (eds.), Academic Press, p 328.

A peptide of 53 amino acids corresponding to the SlpIII sequence was prepared on an Applied Biosystems peptide synthesizer. The yield of this material, which has a molecular weight of 3640 was approximately 0.5 grams. The peptide was coupled to bovine serum albumin. The material was sent to Antibodies, Inc. for preparation of antibodies in rabbits. Antisera was obtained that reacted with synthetic peptides of both the SlpI and SlpIII sequences. These antisera have been useful for the detection of fusion peptides containing gly-ala sequences.

Following the procedure described above an antigen was synthesized having the formula (V-P-G-V-G)$_8$, which was coupled to keyhole limpet hemocyanin. Polyclonal antisera was then prepared as described above which bound to the ELP peptide.

2.    Polyacrylamide gel electrophoresis of proteins:

Approximately $10^9$ E. coli cells from growing cultures were pelleted by centrifugation at 10,000xg for 5 min. The cell pellets were resuspended in 100 to 500 μl of 2X sample buffer (100mM Tris-HCl pH 6.8, 4% SDS, 10% B-mercaptoethanol, 60% glycerol or sucrose) and sonicated for 30 sec using a Tekmar sonic disruptor. Samples were boiled for approximately 5 min and 20 to 100 μl of the cell lysates were loaded on an SDS-polyacrylamide gel (7.5 to 16% w/v). The gels were prepared following the procedure of Laemmli (Nature, 227:680-685 (1970)). The proteins in the gels were stained with 2% Coomassie brilliant blue in 10% methanol, 7.5% acetic acid for 1 hr and destained in 10% methanol, 7.5% acetic acid overnight.

3.    Immunoblotting of proteins in gels:

After protein electrophoresis, one of the flanking glass plates was removed from the polyacrylamide gel. The gel surface was wetted with transfer buffer (25mM Tris-HCl, 192mM glycine, 20% methanol). A piece of nitrocellulose paper (Sartorius, SM11307) was saturated with transfer buffer and laid on the gel. Air bubbles between the filter and the gel were removed. The gel and nitrocellullose filter were placed in the transfer unit as specified by manufacturer (Bio-Rad). Transfer was allowed to proceed at 200 mA for 3-4 hr. Then the nitrocellulose filter was removed and stained with Amido-Schwartz for 3 min (0.05% Amido black, 45% deionized $H_2O$), 45% methanol, 10% acetic acid) and destained in $H_2O$. The filter was incubated for at least 10 min at room temperature in "BLOTTO" (5% w/v nonfat dry milk, 50mM Tris-HCl pH 7.4, 0.9% w/v NaCl 0.2% w/v sodium azide). The filter was placed in serum appropriately diluted (1:50 to 1:500) in 0.5X Blotto (2.5% nonfat dry milk, 50mM Tris-HCl pH 7.4, 0.9% NaCl, 0.2% sodium azide) and was gently agitated for approximately 16 hr at room temperature. The filter was washed for 1 hr with 5 changes of TSA (50mM Tris-HCl pH 7.4, 0.9% NaCl, 0.2% sodium azide). The blot was placed in 15ml of 0.5X BLOTTO solution containing $1x10^7$ cpm of the $^{125}$I -protein A and gently agitated for 2 hr at room temperature. The filter was washed for 2 hr with a minimum of 7 changes of TSA, rinsed once with deionized $H_2O$ and air dried. The blot was covered with Saran wrap and autoradiographed.

4.    Amino Acid Analysis:

Amino acid compositions are determined by the PTC derivitization procedure of Henrickson and Meredith (1984). Protein samples were hydrolysed with 5.7 N constant boiling HCl at 108°C for 24 hours in vacuo. After reaction with PITC, amino acid derivatives were detected at 254 nm by HPLC reverse phase chromatography using a Hewlett Packard 1090 system and a Supelco C18 column (4.6 mm x 25 cm) with a linear gradient of 0-50% acetonitile in 0.1M NH$_4$OAc pH 6.78 as a mobile base. Henrickson, R.L. and Meredith, S.C. (1984) Amino Analysis by Reverse Phase High Performance Liquid Chromatography. Anal. Biochem. 137:65-74.

5.    Amino Acid Sequence Analysis:

The N-terminal amino acid sequence was determined by automated Edman degradation using an Applied Biosystems Model 470A gas phase protein sequenator. The PTH amino acid derivatives were analyzed by reverse phase HPLC using a Hewlett Packard 1090 system and an Altex C18 column (2 mm x 25 cm) with a complex gradient buffer system.

6. Peptide Synthesis:

Synthetic peptides were prepared by solid phase synthesis on an Applied Biosystems Model 430A Peptide Synthesizer using the standard symmetric anhydride chemistry as provided by the manufacturer. The coupling yield at each step was determined by the quantitative ninhydrin procedure of Sarin et al., (1981). The synthetic peptide was cleaved from the solid support and amino acid blocking groups were removed using anhydrous HF (Stewart and Young, 1984). Crude peptides were desalted by chromatography over Sephadex G-50. Sarin, V.K., Kent, S.B.H., Tam, J.P. and Merrifield, R.B. (1981). Anal. Biochem. 237:927-936. Stewart, J.M. and Young, J.D. (1984). Solid Phase Peptide Synthesis, Pierce Chemical Company, Rockford, IL. pp 85-89.

Synthetic DNA Methods

1. In vitro DNA synthesis:

The N,N-diisopropylphosphoramidites, controlled-pore glass columns and all synthesis reagents were obtained from Applied Biosystems, Foster City, California.

Synthetic oligonucleotides were prepared by the phosphite triester method with an Applied Biosystems Model 380A DNA synthesizer using a 10-fold excess of protected phosphoramidites and 1 μmole of nucleotide bound to the synthesis support column. The chemistries used for synthesis are the standard protocols recommended for use with the synthesizer and have been described (Matteucci, et al., Journal Amer. Chem. Soc., 103:3185-3319 (1981)). Deprotection and cleavage of the oligomers from the solid support were performed according to standard procedures as described by McBride, et al., Tetrahedron Letters, 24:245-248 (1983). The repetitive yield of the syntheses as measured by the optical density of the removed protecting group as recommended by Applied Biosystems (1984) was greater than 97.5%.

The crude oligonucleotide mixture was purified by preparative gel electrophoresis as described by the Applied Biosystems protocols of November 9, 1984 (User Bulletin No. 13). The acrylamide gel concentration varied from 10 to 20% depending upon the length of the oligomer. The purified oligomer was identified by UV shadowing, excised from the gel and extracted by the crush and soak procedure (Smith, Methods in Enzymology, 65:371-379 (1980)).

2. Sequencing of DNA:

DNA sequences were determined by the following methods. Fragments containing the region of interest were cloned into the multiple cloning site of M13mp18 or M13mp19 (Maniatis et al., 1982, and Norrander et al., 1983). Single-stranded DNA was prepared and sequenced by the primer extension method (Sanger et al., 1977 and Biggin et al., 1983) using 35S-deoxyadenosine 5'-(alpha-thio)-triphosphate (New England Nuclear) as label. In some cases, reverse transcriptase (Molecular Genetics) was used to extend the primer, using the dideoxy:deoxynucleosidetri-phosphate ratios utilized by Zagursky et al. (Gene Anal. Techn. (1985) 2:89-94). Deoxyadenosine triphosphate labeled with either $^{32}$P or $^{35}$S was used in these reactions. Compression artifacts which appeared in some G-C rich sequences were overcome by eliminating deoxyguanosine triphosphate from the G reaction, and using deoxyinosine triphosphate (P-L Biochemicals) at a final concentration of 37.5 μM instead. In the other mixes, the concentration of dideoxyGTP in the G reaction was 0.5 mM. All sequences were run on 6 or 8% polyacrylamide gels containing 8 M urea (Sanger et al. 1978). Primers used for sequencing were purchased from P-L Biochemicals. Storage and analysis of data utilized software from both DNAstar and International Biotechnologies, Inc.

3. In vitro mutagenesis of cloned DNA:

Plasmid DNA (1 μg) containing the sequence to be mutated was digested in two separate reactions. One reaction contained either one or two restriction endonucleases which cleave at sites immediately flanking the region of interest. In the second reaction, the DNA was digested with a restriction endonuclease which cleaves only once at a site distant from the sequence to be mutated. The DNA fragments generated in the first reaction were separated by agarose gel electrophoresis and the large fragment which lacks the sequence to be mutated was excised and purified. DNA from the second reaction, the large fragment of DNA from the first reaction, and a synthetic oligodeoxy-nucleotide of 20-30 bases in length containing the mutant sequence were mixed in a molar ratio of 1:1:250. The mixture was denatured by heating at 100°C for 3 min in 25 to 100 μl of 100mM NaCl, 6.5mM Tris-HCl pH 7.5, 8mM MgCl$_2$, and

1mM B-mercaptoethanol. The denatured mixture was reannealed by gradually lowering the temperature as follows: 37°C for 30 min, 4°C for 30 min, and 0°C for 10 min. The reaction was supplemented with 0.5mM deoxyribonucleotide triphosphates, 1mM ATP, 400 units of T4 DNA ligase and 5 units of E. coli DNA polymerase large fragment and incubated at 15°C for 12-16 hr. The reaction mixture was then transformed into E. coli and antibiotic-resistant colonies were selected.

## Fermentation Conditions

The fermentor is a 15L Chemap, 10 L working volume. The culture conditions are: temperature = 30°C, pH = 6.8; NaOH 2.5 M is used for pH regulation. The headspace pressure is below 0.1 bar. The dissolved oxygen is regulated at 50%. The air flow varies from 0.5 L/min to 20 L/min. The agitation rate varies between 200 to 1500 rpm.

The fermentor is inoculated with a 10% (v/v) inoculum grown in medium A for 15 hours at 30°C under agitation.

Medium B was the fermentor medium. The starting volume was 5 L.

When the glucose concentration reached 1%, a concentrated solution (5x) of medium B was added to the fermentor in order to keep the glucose concentration approximately at 1%. When the culture reached an $OD_{600}$ of 60.0, the temperature was increased to 42°C for 10 min, then lowered to 39°C for 2.5 hours. The cells were then harvested by centrifugation and frozen at -70°C until processed.

Table 1

| Medium A: | LB Medium |
|---|---|
| Constituent | g/L |
| NaCl | 10 |
| tryptone | 10 |
| yeast extract | 5 |
| kanamycin | $5x10^{-3}$ |
| Medium B | |
| Constituent | g/L |
| $NH_4Cl$ | 4.5 |
| $KH_2PO_4$ | 0.76 |
| $MgSO_4 \cdot 7H_2O$ | 0.18 |
| $K_2SO_4$ | 0.09 |
| $CaCl_2$ | $24x10^{-3}$ |
| $FeSO_4 \cdot 7H_2O$ | $7.6x10^{-3}$ |
| TE | 0.5 ml |
| casamino acids | 25 |
| yeast extract | 5 |
| glucose | 20 |
| kanamycin | $5x10^{-3}$ |

## Example 2

## Assembly and Expression of the SlpI Gene

1.   Summary of the scheme for assembling the SlpI gene:

An 18 bp DNA sequence that codes for the most frequent repeating oligopeptide in the silk fibroin protein made by Bombyx mori [Lucas, F. and K.M. Rudall (1986) Extracellular Fibrous Proteins: The Silks. p 475-558, in Comprehensive Biochemistry, vol. 26, part B., M. Florkin and F.H. Stotz (eds.) Elsevier, Amsterdam] was synthesized in vitro. Two single-strands were synthesized, annealed together and then the resulting double-stranded segments were multimerized head-to-tail to generate concatamers of up to and exceeding 13 repeats. The structural gene for silk I that we proceeded to work with had 13 repeats that coded for the oligopeptide gagags, where g = glycine, a = alanine and s = serine. We refer to this structural gene as the "monomer". We constructed "dimeric, trimeric, tetrameric, pentameric and hexameric" SlpI genes containing 26 (SlpI-2), 39 (SlpI-3), 52 (SlpI-4), 65 (SlpI-5) and 78 (SlpI-6) repeats. There is a short intervening sequence between each monomer unit. The assembly is pictured as follows:

Repeating DNA sequence 5'-G G T G C G G G C G C A G G A A G T
C G C C C G C G T C C T T C A C C A-5'

"Monomer"

Multimers

### 2. Assembly of the "monomeric" SlpI structural gene:

The two single-strands shown above were synthesized as previously described. The strands were separately purified by gel electrophoresis, phosphorylated using T4 polynucleotide kinase and then mixed together and allowed to anneal. This resulted in the double-stranded segments aligning spontaneously head-to-tail in long concatamers. The phosphodiester bonds between segments were formed with T4 DNA ligase. The reaction was stopped by filling in the terminal cohesive ends using the Klenow fragment of DNA polymerase I. The blunt-ended repeating DNA was then ligated to the HincII REN site in plasmid vector pUC12 (Veiera, et al., Gene 19:259-268 (1982)). The ligated DNA was transformed into E. coli HB101 and transformants were selected for their ability to grow in the presence of ampicillin. The DNA of potential clones was analyzed; for size and orientation by REN digestion and gel electrophoresis. DNA sequences were determined for isolates with large inserts that were oriented properly. The "monomer" clone selected for subsequent multimerization had 13 repeats coding for the oligopeptide agagsg, and was named pSY708. The DNA sequence, deduced amino acid sequence and REN sites of the SlpI insert and flanking regions of pSY708 are shown in Table 2.

## Table 2

```
H                P      A S
I                S      V M
N                T      A A
3                I      I I
:                :      : :
AAGCTTGGGCTGCAGGTCACCCCGGCGGGCGCAGGAAGTGGTGCGGGCGCAGGAAGTGGT
----.----+----.----+----.----+----.----+----.----+----.----+  60
TTCGAACCCGACGTCCAGTGGGGCCGCCCGCGTCCTTCACCACGCCCGCGTCCTTCACCA

k  l  g  l  q  v  t  r  a  g  a  g  s  g  a  g  a  g  s  g
----.----+----.----+----.----+----.----+----.----+----.----+


GCGGGCGCAGGAAGTGGTGCGGGCGCAGGAAGTGGTGCGGGCGCAGGAAGTGGTGCGGGC
----.----+----.----+----.----+----.----+----.----+----.----+  120
CGCCCGCGTCCTTCACCACGCCCGCGTCCTTCACCACGCCCGCGTCCTTCACCACGCCCG

a  g  a  g  s  g  a  g  a  g  s  g  a  g  a  g  s  g  a  g
----.----+----.----+----.----+----.----+----.----+----.----+


GCAGGAAGTGGTGCGGGCGCAGGAAGTGGTGCGGGCGCAGGAAGTGGTGCGGGCGCAGGA
----.----+----.----+----.----+----.----+----.----+----.----+  180
CGTCCTTCACCACGCCCGCGTCCTTCACCACGCCCGCGTCCTTCACCACGCCCGCGTCCT

a  g  s  g  a  g  a  g  s  g  a  g  a  g  s  g  a  g  a  g
----.----+----.----+----.----+----.----+----.----+----.----+


AGTGGTGCGGGCGCAGGAAGTGGTGCGGGCGCAGGAAGTGGTGCGGGCGCAGGAAGTGGT
----.----+----.----+----.----+----.----+----.----+----.----+  240
TCACCACGCCCGCGTCCTTCACCACGCCCGCGTCCTTCACCACGCCCGCGTCCTTCACCA

s  g  a  g  a  g  s  g  a  g  a  g  s  g  a  g  a  g  s  g
----.----+----.----+----.----+----.----+----.----+----.----+


X                B      A S              E
B                A      V M              C
A                M      A A              R
I                I      I I              I
:                :      : :              :
GCGGGCGCAGGAAGTGGCACTCTAGAGGATCCCCCGGGCGAGCTCGAATTC
----.----+----.----+----.----+----.----+----.----+  290
CGCCCGCGTCCTTCACCGTGAGATCTCCTAGGGGGCCCGCTCGAGCTTAAG

a  g  a  g  s  g  t  l  e  d  p  r  a  s  s  n  s
----.----+----.----+----.----+----.----+----.----+
```

3.    Construction of the expression vector, pSY701:

Plasmid pSP65 (10 μg, Boehringer Mannheim) was digested with AatII REN, phenol extracted and ethanol precipitated. The DNA was resuspended in 10μl of H$_2$O. One-half of this DNA was digested with exonuclease III in the following mix: 5 μg DNA, 10 μl 10X exonuclease III buffer (600mM Tris-HCl pH 8.0, 6.6mM MgCl$_2$, 10 mM β-mercap-

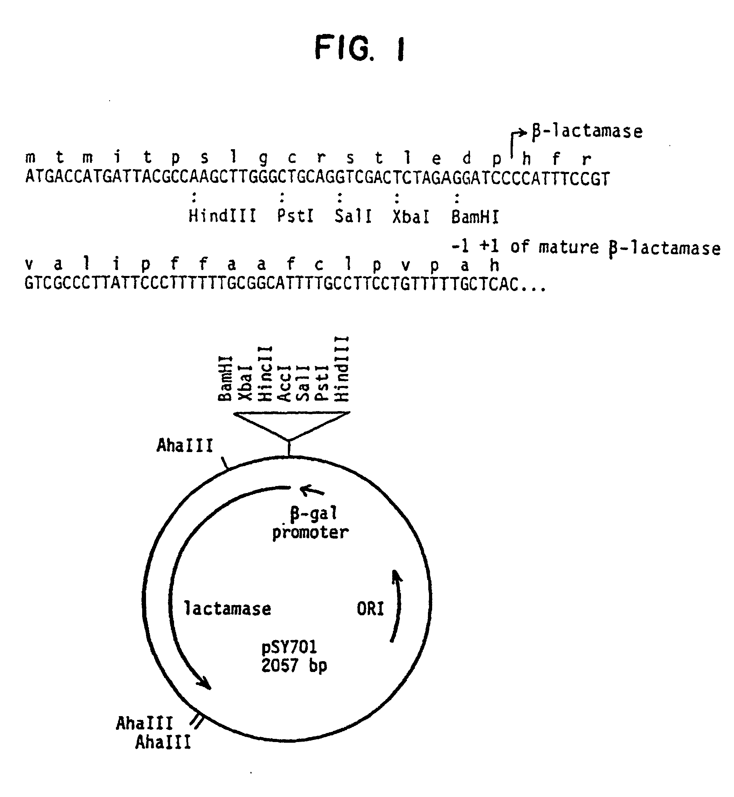

toethanol) and 9 units of exonuclease III in a total volume of 200 μl. Samples of 20 μl were taken at 0, 1, 2.5, 5 and 7.5 min and diluted immediately in 100 μl of the following buffer (30 mM sodium acetate, pH 4.5, 0.25 M NaCl, 1mM $ZnSO_4$) containing 5 μg tRNA and 36 units of S1 nuclease. Incubation was at 30°C for 45 min and then the reaction was terminated by the addition of 15 μl of stop buffer (0.5M Tris pH 9.0, 125mM EDTA, 1% w/v SDS, 200 μg/ml tRNA). The samples were phenol extracted and ethanol precipitated. The resuspended DNA was digested with SmaI REN and electrophoresed through a 1% gel of low melting point agarose. The gel band corresponding to the DNA fragment carrying the β-lactamase gene, the plasmid origin and the β-galactosidase promoter was excised from the gel and melted at 65°C. One volume of $H_2O$ was added. The DNA in each sample (timepoint) was recircularized by ligation in the presence of agarose. The reaction included 8 μl melted gel, 2 μl of ligation buffer (100mM Tris-HCl pH 7.5, 50mM $MgCl_2$, 50mM DTT, 1mM ATP), 10 units T4 DNA ligase and was incubated at 15°C for 3 hr. Competent cells of JM101 were transformed with the ligated DNA and transformants were selected by growth on L broth plates containing ampicillin (40 μg/ml). Plasmid DNA was prepared from four transformants. The DNA was digested with BamHI REN, labeled with $^{32}$P-dGTP using the Klenow fragment of DNA Polymerase I, digested with Pvu I and then the smallest fragment was gel purified. The fragment from one transformant was sequenced using the Maxam and Gilbert technique. The fragments of the other three plasmids were further digested with TaqI and electrophoresed on the same gel. The sequenced plasmid had a fusion between the multiple cloning site and a position upstream from the N-terminal ATG of β-lactamase. The size of the BamHI-TaqI fragment of two of the other plasmids indicated a fusion between the multiple cloning site and the 4th amino acid of the β-lactamase gene. The DNA and corresponding amino acid sequences of the N-terminal region of the altered β-lactamase are given below, along with a circular map of REN sites for pSY701 (see Figure 1). The amino acid sequence of Figure 1 is met-thr-met-ile-thr-pro-ser-leu-gly-cys-arg-ser-thr-leu-glu-asp-pro-his-phe-arg-val-ala-leu-ile-pro-phe-phe-ala-ala-phe-cys-leu-pro-val-phe-ala-his.

### 4. Insertion of "monomer" SlpI from pSY708 into pSY701:

Plasmid pSY708 was digested with HindIII, the cohesive ends were filled in using the Klenow fragment of DNA polymerase I and then digested with BamHI. Plasmid pSY701 was digested with XbaI, filled in as above and then digested with BamHI. The DNA fragment from pSY708 and the backbone of pSY701 were then purified by electrophoresis through a low melting temperature agarose gel and purified with NACS (BRL) columns. The appropriate fragments were mixed, ligated, and then transformed into E. coli JM109. Transformed cells were selected by growth on L plates containing ampicillin (40 mg/ml), IPTG ($5 \times 10^{-4}$M) and XGAL (20 mg/ml). Transformants were analyzed for plasmid contents and one (pSY756) was selected for further study since it carried the insert of the monomer SlpI-1 sequences in the proper orientation, as determined by mapping of REN sites. Although the entire DNA sequence was not determined for pSY756, the junctions between the insert and vector were verified as correct restriction sequences for XbaI, upstream and BamHI, downstream.

### 5. Multimerization of the SlpI gene of pSY756:

Plasmid pSY708 was digested with the REN SmaI and the DNA fragment carrying the coding sequence for the polypeptide arg(ala-gly-ala-gly-ser-gly)$_{13}$thr-leu-glu-asp-pro (R(AGAGSG)$_{13}$TLEDP) was purified as in 4 above. Plasmid pSY756 was digested with SmaI, de-proteinized and then ligated with the purified DNA fragment from pSY708. Transformants of E. coli JM109 were selected on medium containing ampicillin. Clones were found to contain 2 units (dimer pSY882), 3 units (trimer pSY883), and 4 units (tetramer pSY915) of the original monomer sequence of the pSY708 clone. Similarly, pentamers and hexamers have also been constructed. All of these plasmids are genetically stable and produce the gly-ala peptide as a fusion with β-lactamase.

### 6. Expression of the SlpI gene fusion to the β-lactamase protein:

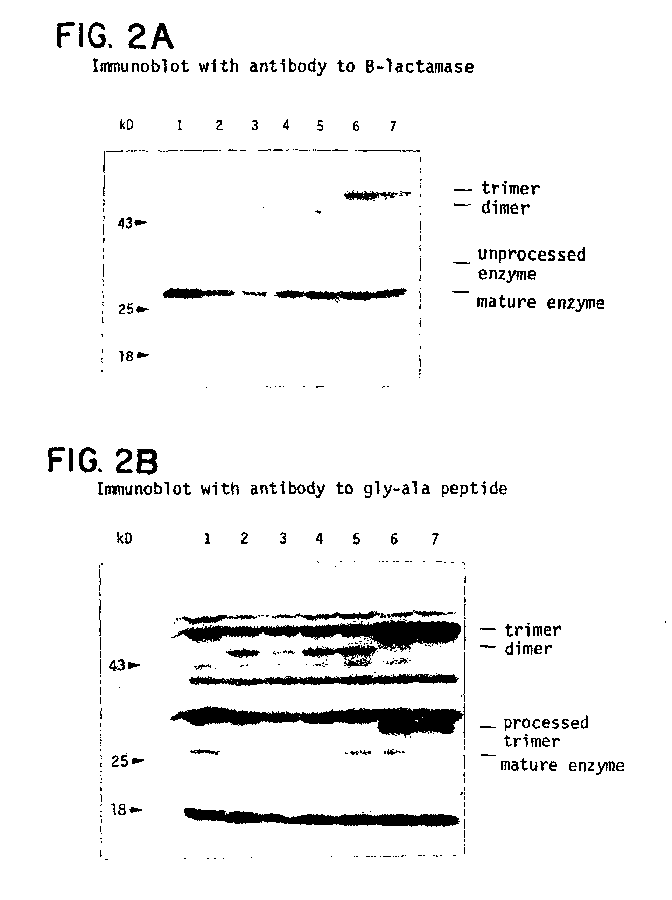

Synthesis in E. coli cells of the SlpI peptide as a fusion protein with β-lactamase was detected by immunoblotting (Western) analysis. Anti-"Slp" antibodies were raised against a synthetic silk peptide. Fusions between β-lactamase and SlpI were also detected with antibodies raised against the E. coli β-lactamase. As shown in Figure 2, this antibody reacts with dimers and trimers of SlpI fused to the E. coli β-lactamase. The SlpI insert proceeds the fifth amino acid of the signal sequence for this enzyme. The β-lactamase antibody (Figure 2A) detects both the unprocessed fusion proteins as well as the processed mature enzyme which appears as the major antigenic band in this figure, at about the 28 kD position. The mobilities of all Slp-containing polypeptides are anomalously slow and the proteins are not as large as they appear on the gels.

The anti-Slp antibody also is useful in detecting these fusion products. Lanes 2-5 of Figure 2B represent 4 separate clones that contain dimer fusions of SlpI with β-lactamase, while lanes 6 and 7 are from two clones containing trimer fusions. As can be seen the antigenicity of the trimer is considerably greater than for the dimer. It is known from prior experiments that fusion proteins containing only a monomer of SlpI are not detected at all with this anti-Slp antibody. The increased antigencity of the trimer peptide allows it to be detected as a processed fusion with the β-lactamase signal peptide. The processed form is seen at about the 33 kD position in lanes 6 and 7 of Figure 2B. The appearance of normally processed β-lactamase mature enzyme (detected with β-lactamase antibody) as well as a peptide corresponding to the fusion between the SlpI-3 trimer and the signal peptide of β-lactamase (detected with gly-ala antibody) suggests that despite the insertion of SlpI sequences within the signal sequence, normal proteolytic processing of the enzyme occurs in E. coli.

7.a.     Expression of the SlpI gene by fusion to T7 genes:

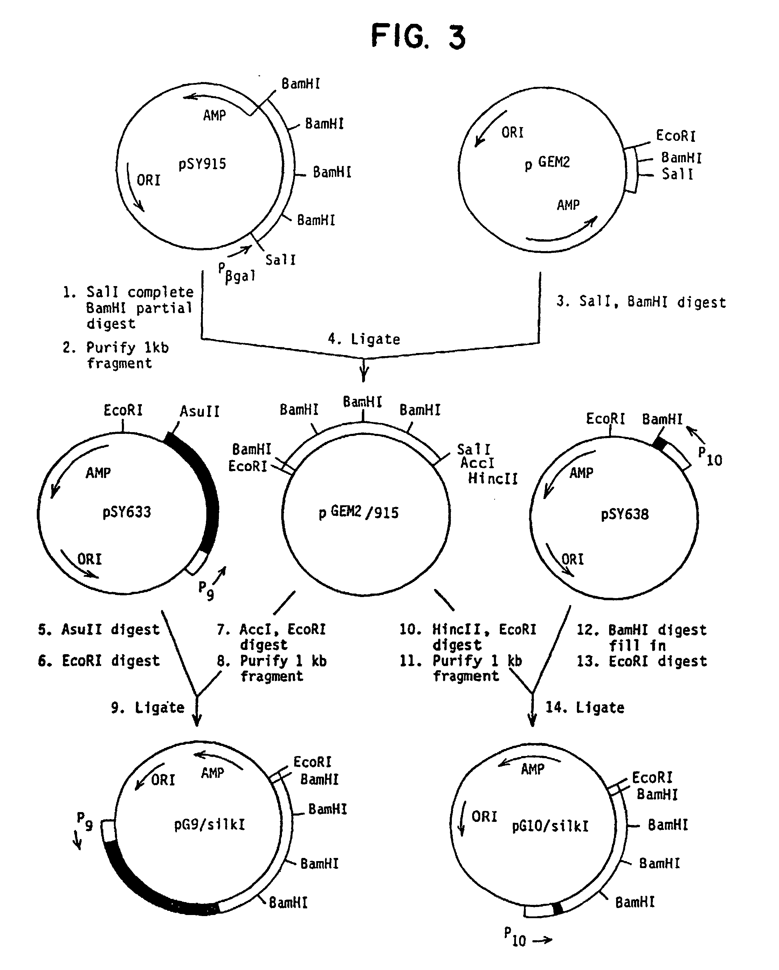

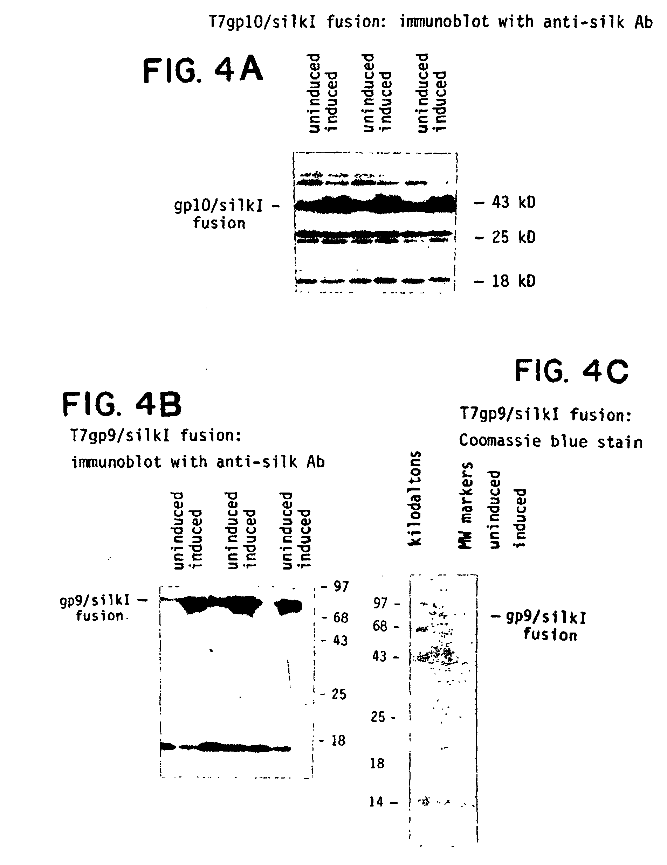

The SlpI sequence has also been expressed as a fusion protein with both the gene 9 and gene 10 proteins from bacteriophage T7 in E. coli. The construction is diagrammed in Figure 3. Plasmid pSY915 (containing the SlpI-4 tetramer) was digested to completion with REN SalI and partially with BamHI. The DNA fragment containing the SlpI-4 tetramer was purified and then cloned in plasmid pSY114 (pG2 of Promega Biotech) which had been digested with RENs SalI and BamHI. From this intermediate plasmid, the tetramer insert of SlpI was removed with the RENs AccI and EcoRI. This fragment was then cloned in pSY633 (pBR322 containing the complete T7 gene 9 sequence; pAR441 of Studier et al., (1986)) which was digested with EcoRI and AsuII. In the resulting plasmid the SlpI tetramer is fused to the gene 9 translational reading frame near the C-terminus of gene 9. This plasmid was then used to transform E. coli strain 0-48 (strain HMS174 (λDE3) of Studier, et al., 1986) which contains the T7 RNA polymerase gene inserted into the chromosome under transcriptional control of the IPTG-inducible β-galactosidase promoter. In this configuration, expression of the SlpI-4 sequence is dependent upon production of the T7 RNA polymerase which itself is controlled by the IPTG-inducible β-galactosidase promoter. As shown in Figure 4B and 4C, when these cells are induced with IPTG a protein product of the gene 9/SlpI-4 fusion gene is synthesized and is detected with antibody to the synthetic Slp peptide. The fusion product migrates in the gel as if it was 82 kD in size. The size expected is only 65 kD. The anomalous mobility is characteristic of the unusual amino acid composition (rich in glycine and alanine) and is seen for all Slp-containing products.

In like manner, plasmid pSY638 (pAR2113 of Studier) containing the promoter region and the first 13 amino acids of the T7 gene 10 protein, was digested with REN BamHI, filled in with the Klenow fragment of DNA polymerase and then digested with REN EcoRI. Into this linearized plasmid was cloned the AsuII-EcoRI fragment of pSY633, containing the SlpI-4 tetramer. This ligation creates an in-frame fusion of the silk tetramer following the thirteenth amino acid of T7 gene 10. The latter fusion product may be used for spinning without further processing since the N-terminal 13 amino acids are only a small part of the large SlpI protein. Although the fusion product is about 30 kD in size, it has an anomalous mobility and migrates as if it was larger, 50 kD. This is shown in Figure 4A.

The plasmids pG9/SlpI-4 and pG10/SlpI-4 were further improved by inserting a kanamycin-resistance gene in the β-lactamase gene in the orientation opposite to the T7 expression system. Thus, any low level expression from the T7 system does not lead to elevated β-lactamase activity. Such activity elminated the ampicillin in the medium that was added to select for maintenance of the plasmid. When the ampicillin was depleted the plasmids were lost from the culture. The kanamycin-resistance gene circumvents this problem and represents a significant improvement in the T7 expression system, especially for large scale cultures. The kanamycin-resistance gene (originally from Tn903) was isolated from a plasmid pUC4K (Veira, J. and J. Messing, 1982. Gene. 19:259-268) was a HincII fragment.

7.b.     Fermentation and purification of SlpI-4:

E. coli strain 0-48 carrying pSY997 was grown at 37°C, using a Chemap or a Braun fermentor, in 10L of LB to an OD (Klett units) of 300 ($3\times10^9$ cells/ml). The T7 system was then induced with the addition of 3.5 mM IPTG. After 150 min the cells were concentrated 10x using a Millipore filter unit (Pellicon cassette system, 100,000 molecular weight cut off filter). The cell suspension was then frozen at -70°C until processing.

The cell suspension was melted in a water bath at 42°C and lysed in a french press, and the lysate was spun at 125,000xg for 1 hour at 25°C. The cleared supernatant was treated with DNAase (250 μm/ml) for 15 min at room temperature, then filtered through a .45 μm sterile filter. The filtrate volume was measured and incubated in ice with slow stirring. Then 231 mg of ammonium sulphate were added for each ml of filtrate over a period of 45 min. One ml of NaOH for each 10 g of ammonium sulphate was added to neutralize the pH. After 2 hours of continuous stirring the mixture was spun at 9,000xg for 10 min. The pellet was resuspended in 1/10 of the original filtrate volume using distilled water. The centrifugation and resuspension was repeated three times. The pellet was resuspended in 1/10 of the original filtrate volume in distilled water. Samples were analyzed for protein concentration, amino acid composition and protein sequence by standard methods. This is one of several methods for obtaining the product. This method results in an SlpI-4 product that is greater than 90% pure. The amino acid composition is almost entirely gly, ala and ser, as expected, and the N-terminal amino acid sequence is that of the gene 10 leader.

8.    Controlled expression of the T7 RNA polymerase gene in Bacillus subtilis:

The coding sequence of the T7 RNA polymerase gene (T7 gene 1, T7 nucleotides 3128 to 5845) from plasmid pSY558 (pAR1151 of Studier, et al., 1986) was modified by in vitro mutagenesis of cloned DNA. We inserted the recognition sequence for the restriction endonuclease NdeI at position 3171. Using an oligodeoxynucleotide which was synthesized as previously described, the T7 gene 1 sequence was changed from its natural sequence, TAAATG, to the modified sequence, CATATG.

Similarly, the upstream regulatory sequence of the Bacillus subtilis gene spoVG, obtained from plasmid pCB1291 (Rosenblum, et al., J. Bacteriology, 148:341-351 (1981)), was modified by in vitro mutagenesis at position 85 (Johnson, et al., Nature, 302:800-804 (1983)) such that it also includes an NdeI cleavage site. The upstream regulatory sequences of the spoVG gene were then ligated with the coding sequence of the T7 RNA polymerase gene via these novel NdeI cleavage sites. After transformation of E. coli HB101, the plasmid contents of individual ampicillin-resistant isolates were checked by restriction mapping. The correct construction was named pSY649.

Plasmid DNA containing the spoVG:T7 RNA polymerase fusion gene (pSY649) was further modified to include a chloramphenicol-resistance gene that functions in B. subtilis. First the NdeI to SalI fragment of about 1200 base pairs from plasmid pGR71-P43 (Goldfarb, et al., Nature, 293:309-311 (1981)) was isolated. This fragment carries the P43 promoter of B. subtilis and an adjacent chloramphenicol acetyltransferase gene from Tn9. After filling in all the cohesive ends using the Klenow DNA polymerase reaction, this fragment was inserted into the XbaI site within the multiple-cloning site of pUC13 (Veiera, et al., Gene, 19:259-268 (1982)). Ampicillin and chloramphenicol-resistant transformants were selected for further use. The correct plasmid construction was named pSY630. The SmaI to HincII endonuclease cleavage fragment from plasmid pSY630 containing the chloramphenicol acetyltransferase gene fused to the P43 promoter sequence was gel purified and blunt-end ligated to the PvuI site of plasmid pSY649 that had been treated first with T4 DNA polymerase. The resulting plasmid, pSY856, was then transformed into B. subtilis I168. Because plasmid pSY856 is unable to replicate autonomously in B. subtilis, stable transformants resistant to chloramphenicol must result from the integration of the plasmid into the B. subtilis chromosome (Ferrari, et al., J. Bacteriology, 154: 1513-1515 (1983)). The integration event, facilitated by homologous recombination, most likely occurred at either the spoVG or the P43 loci of the bacterial chromosome (pSY856 contains DNA sequences homologous to the B. subtilis chromosome at only these two sites). The resulting strain, "BIPoL", was grown both in the presence and absence of chloramphenicol in order to determine the stability of the selectable marker. Expression of the T7 polymerase was obtained and this has no apparent effect on the growth or viability of this strain.

9.a.    Expression of a plasmid-borne target gene (kanamycin-resistance) in B. subtilis strain BIPoL:

The Staphylococcus aureus plasmid pUB110 (Lacey, et al., J. Med. Microbiology, 7:285-297, 1974) which contains the gene coding for resistance to the antibiotic kanamycin was used to test the expression of the growth-regulated spoVG:T7 RNA polymerase gene of strain B1PoL. An EcoRI-BamHI fragment of phage T7 DNA (positions 21,402 to 22,858 containing the T7 gene 9 promoter sequence was purified from plasmid pAR441 (Studier, et al., 1986). This DNA fragment was ligated into pUB110 between the EcoRI and BamHI restriction endonuclease sites. The resulting plasmid, pSY952, contains the T7-specific promoter in the same orientation as the kanamycin-resistance gene. Plasmid pSY952 was transformed into B. subtilis I168 and BIPoL and these strains were analyzed for the level of expression of the polypeptide encoded by the plasmid-derived kanamycin-resistance gene. Approximately $10^9$ cells from growing cultures of I168, I168 containing pUB110, 1168 containing pSY952, BIPoL, BIPoL containing pUB110, and BIPoL containing pSY952 were obtained at several times during the growth and sporulation cycle. The proteins in these cell samples were processed and analyzed by polyacrylamide gel electrophoresis.

Because the rate of transcription from the spoVG promoter increases as a function of cell density and reaches a maximum during early sporulation, an accelerated accumulation of the target protein is expected in the BIPoL strain containing pSY952 during growth as the culture enters sporulation. The results show that a protein of molecular weight 34 kilodaltons increases in abundance as the culture approaches and enters stationary phase. The size of the protein is in agreement with the predicted size of the kanamycin-resistance gene product (Sadaie, et al., J. Bacteriology, 141: 1178-1182 (1980)) encoded in pSY952. This protein is not present in B1PoL or 1168 containing pSY952 which lacks the spoVG-regulated T7 RNA polymerase gene or in B1PoL containing pUB110 which lacks the T7 promoter sequence. The maximum accumulated level of target protein after 24 hours of growth in B1PoL containing pSY952 was 20% of the total cellular protein as determined by densitometry.

9.b    Expression of SlpI-4 in B. subtilis:

Plasmid pG10SlpI was digested with EcoRI REN. After filling in the cohesive ends using the Klenow DNA polymerase reaction, the DNA was digested with Bg1II REN. Plasmid pSY662 was digested with SmaI and BamHI RENs. The two plasmids were then purified by electrophoresis through a low melting temperature agarose gel and purified with NACS (BRL) columns. The DNA fragment of pG10SlpI was ligated to the backbone of pSY662 and transformed into E. coli containing ampicillin (40 μg/ml). Transformants were analyzed for plasmid contents and one (pSY662/G10/SlpI-4) was selected for further study.

Competent cells of B. subtilis BIPol were transformed with pSY662/G10/SlpI-4 and incubated at 37°C with shaking for 90 min. The transformation mixture was then diluted 1:100 in fresh LB containing 10 μg/ml of tetracycline and incubated at 37°C with shaking. Samples were taken and equal numbers of cells were lysed and loaded on gels for separation by SDS-PAGE. Immunoblot analysis was performed using anti-Slp antibodies to detect the synthesis of the gene 10/SlpI-4 fusion protein.

The expression of the SlpI-4 polypeptide in B. subtilis was detected by its seroreactivity with anti-Slp antibody, after transfer of the cellular proteins from the polyacrylamide gel to a nitrocellulose filter. We verified that the seroreactive protein was the product of the SlpI-4 gene by exhaustively treating the cellular proteins with CNBr. This should cleave after methionine residues, but since SlpI-4 lacks methionine it will remain intact. The CNBr treatment eliminated greater than 98% of the proteins stainable with Coomassie blue dye. And as expected for a protein lacking methionine, SlpI-4 remained intact and still reacted with anti-Slp serum.

Example 3

Assembly and Expression of the SlpIII Gene

    1.    Summary of the scheme for assembling the SlpIII gene:

The synthetic SlpIII gene codes for a protein similar to the SlpI gene and to the crystalline region of the silk fibroin protein made by the silkworm, Bombyx mori. SlpIII more closely resembles the silk fibroin molecule because it includes the amino acid tyrosine at regular intervals (about 50 residues), whereas multimers of SlpI do not. The SlpIII gene was assembled from smaller parts. First, three double-stranded sections of DNA of about 60 bp in length were chemically synthesized. Each section was cloned by insertion into bacteriophage M13 and the DNA sequence was verified. These sections were then removed from the vector and linked together in a specific order. This linkage of about 180 bp is named the SlpIII "monomer". "Monomers" were then linked in a specific order to yield dimers, trimers, tetramers, etc., of SlpIII. The multimers were then cloned either directly into plasmid expression vectors to detect the SlpIII protein or initially into an adapter plasmid. Insertion of the SlpIII DNA into the adapter allows for further gene manipulation and is further described later. The assembly scheme is pictured as follows:

Synthesis of double-stranded DNA sections --

Section 1 -

Section 2 -

Section 3 -

Assembly of "monomer" --

Multimerization --

The DNA and corresponding amino acid sequences of the three sections of the SlpIII gene are shown in Table 3.

**Table 3**

Section 1 (61 bds / 15 bds):

```
      X   H      Sma I
GGT GGC AGC GGT GCA GGA GCC GGT GGC GGC TCT GGA GCT GGC GCG GGC CCT CCT AG
CCA CCG TCG CCA CGT CCT CGG     ...  AGA CCT CGA ...        ...
 G   G   S   G   A   G   A   G   G   G   S   G   A   G   A   G
```

Section 2 (68 bds / 60 bds):

```
BamHI                                        Pst I  GCA GCT GCG
GA TCC GGC GCA GGC GGT TCT GGC GCA GGC GGC TCT GGC GCA GGA GGG GCT CGT CGG TTC GGA CCT CGT CCT CCG AGA CCT CGG
G    G   S    G    A   G   S    G    A   G   S    G   A   G    S
```

Section 3 (55 bds / 43 bds):

```
                    X   H   d    Hind III  GCA GCG GCG GCC A
A CGT CCG ATA CCT CGA CCT CGA CCG AGT CCA CGG GCA AGC AGA GCG GCG GCT GGA GGT GGC TCA GGT GCT GGC GCT GGC TAT GGA GCT GGC
 A   G   S   S   G   A   A   G   A   G   A   G   S   G   A   G   A   G   Y   G   A
```

The double-stranded DNA sequence is shown in the 5' to 3' direction. The amino acids (g = glycine, a = alanine, s = serine, y = tyrosine, coded by the sequence are shown immediately below each section. Recognition sequences for cleavage by restriction endonucleases are shown above each section.

The above six single-strands were synthesized. After synthesis, the strands of DNA were purified and the homologous strands were annealed. About 1 µl (0.5 µg) of each strand was mixed with 2 µl of 10X AA (description) buffer and 16 µl of sterilized deionized $H_2O$ in a 1.5 ml polypropylene Eppendorf tube. The tube was placed in a boiling water bath (500 ml in a 1 liter beaker) for 10 min and then the beaker was removed from the hot plate and allowed to cool on the bench to room temperature. This required about 1-2 hr.

Each of the three double-stranded sections was cloned separately into M13mp18. Section 1 was ligated between the Sma I and BamHI restriction sites of the multiple-cloning site. Section 2 was ligated between the BamHI and PstI sites. And section 3 was inserted between the PstI and HindIII sites. The respective clones are: M13mp18.1, M13mp18.2, M13mp18.3. The DNA sequence was determined for each cloned section. One representative of each section that had the correct DNA sequence was recovered and became the material for the next step: assembly of the "monomer".

3.    Assembly of the "monomer" of SlpIII:

The DNA sections 2 and 3 were isolated by digestion of the M13 clones with restriction enzymes: for section 2, M13mp18.2 was digested with BamHI and Pst1; for section 3, M13mp18.3 was digested with Pst1 and HindIII. The two sections were purified and mixed together in equal molar amounts with M13mp18.1 that had been first digested with BamHI and HindIII. T$_4$ DNA ligase was added to link the homologous overlapping ends in the order 1-2-3. Due to the hybridization specificity of the cohesive ends, the three sections are efficiently linked in only this order. The DNA sequence of the cloned "monomer" in the assembly named M13mp18.1.2.3 was determined to be correct and as shown in 2 above.

4.    Multimerization of the "monomer" of SlpIII:

In order to prepare large amounts of the "monomer" structural gene we first subcloned the "monomer" into the plasmid vector pUC12, M13mp18.1.2.3 was digested with EcoRI and HindIII restriction enzymes. The SlpIII "monomer" was gel purified and ligated into pUC12 digested with EcoRI and HindIII. The resulting plasmid DNA was prepared, the "monomer" was released from the vector by digestion with BanI REN and the fragment was gel purified.
To create multimers, "monomer" DNA with BanI ends were linked by ligation. The nonpalindromic terminal BanI recognition sequence allows linkage only in a head-to-tail order. The extent of multimerization is monitored by gel electrophoresis and staining the DNA with ethidium bromide. Multimers of more than 20 units have been obtained by this method.

5.    Cloning of the multimers of SlpIII:

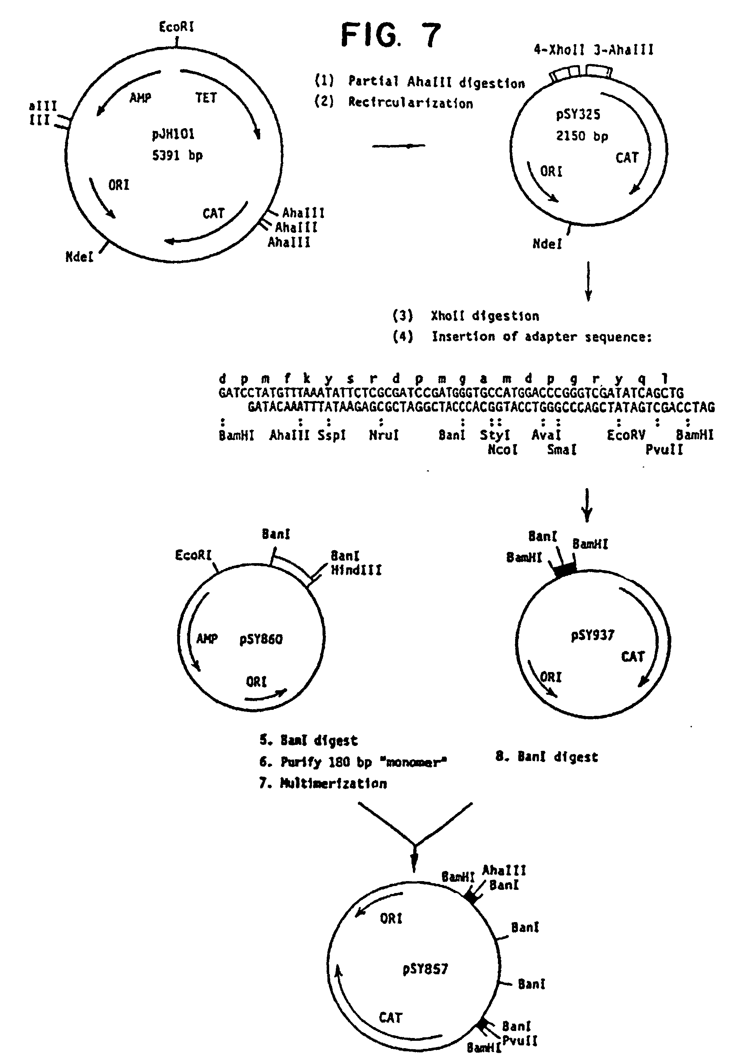

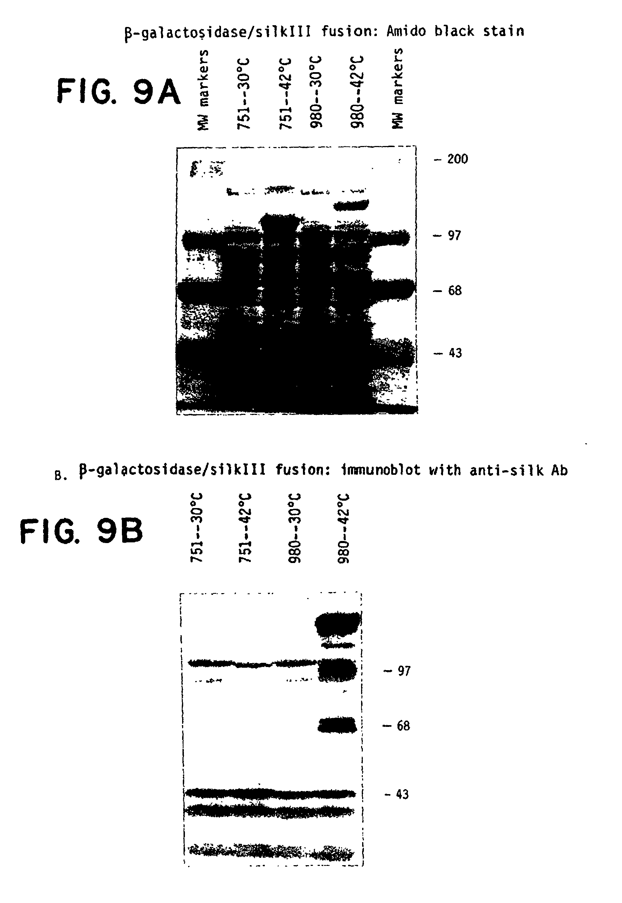

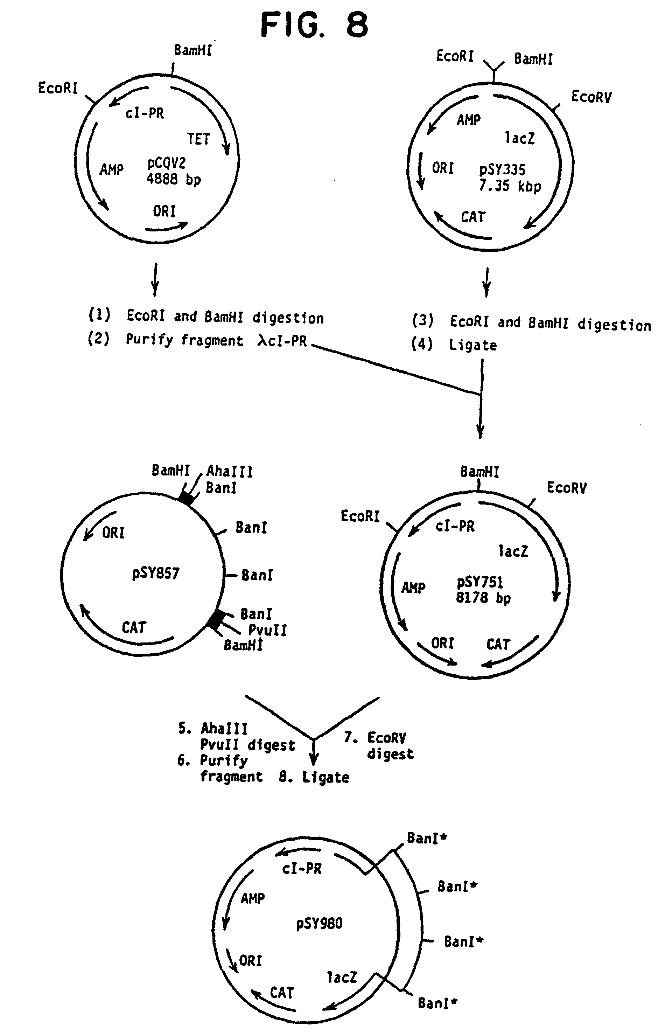

Plasmid pCQV2 (Queen, et al., J. Appl. Mol. Gen., 2:1-10 (1983)) was digested with EcoRI and BamHI restriction endonucleases and a fragment of about 900 bp was purified. This DNA fragment contains the bacteriophage lambda cI-857 repressor gene, the closely linked rightward promoter, P$_R$, and the beginning of the cro gene. Plasmid pSY335 (described as pJF751 in Ferrari, et al., J. Bacteriology, 161:556-562 (1985)) was digested with EcoRI and BamHI restriction enzymes and subsequently ligated to the DNA fragment of approximately 900 bp of pCQV2. The plasmid obtained from this construction, pSY751, expresses the β-galactosidase gene at 37°C and 42°C, but not at 30°C (Figure 8).
In this approach the SlpIII gene is first cloned into an "adapter" sequence in an intermediate plasmid and then subcloned to the expression systems. The adapter sequence has the following useful features: a unique central BanI REN site, three unique REM sites to either side of BanI, information coding for protein cleavage at either methionine, aspartate-proline or arginine amino acids and small size. The BanI site is the point of insertion for the SlpIII multimers with BanI ends.
The adapter was synthesized with the Applied Biosystems 380A Synthesizer, cloned in M13mp18 and the DNA sequence verified. The adapter was then subcloned into a specially-constructed plasmid vector that lacked BanI REN sites. The recipient plasmid was made as follows. Plasmid pJH101 (Ferrari, et al., 1983) was partially digested with AhaIII restriction enzyme and religated. Transformants of E. coli HB101 were selected on medium containing chloramphenicol (12.5 mg/ml). After restriction analysis of several isolates one plasmid was chosen, pSY325 (Figure 7). This plasmid contains only the chloramphenicol-resistance gene and the replication origin (from pBR322) of pJH101. After digestion to completion with XhoII, pSY325 was ligated with the gel-purified adapter. The result was the adapter-plasmid, pSY937, and its new REN sites were verified.
The SlpIII multimers were cloned into the BanI site of pSY937 (Figure 7). Positive clones were identified by colony hybridization and with the lower strand of section 1 of SlpIII as the DNA probe for hybridization (probe sequence shown in Table 2). Positive clones were characterized by gel electrophoresis for the size of the inserted multimer. Finally, the SlpIII sequences were subcloned using the REN site in the flanking adapter regions to specific locations of expression plasmids.
The SlpIII protein had the following amino acid composition:

```
SlpIII      1178 AA         MW 83,000


(fm)    DPVVLQRRDWENPGVTQLNRLAAHPPFASDPM
        GAGS (GAGAGS)6 GAAGY
        [(GAGAGS)9 GAAGY]18
        GAGAGSGAGAGSGAGAMDPGRYQLSAGRYHYQLVWCQK
```

(fm) intends the initiation codon

SlpIII Expression Vector

Plasmid DNA pSY1086 is a pSY937 derivative containing 19 repeats of SlpIII (3.5 kb). This plasmid DNA was digested with Nrul and Pvull and the fragments separated by agarose gel electrophoresis. The purified SlpIII multimer was then cloned in plasmid pSY751 digested with Pvulll REN. Several clones were analyzed and one (pSY1008) was chosen to be used in expression experiments and SlpIII purification.

The ampicillin drug resistance gene of pSY1008 was substituted with the Kanamycin marker from pSY1010 (produced by digestion of pSY633 with Dral and Sspl and insertion of $Kan^R$ obtained by HincII digestion of pUC4K) and the subsequent plasmid was called pSY1186. By removing the SlpIII portion of plasmid pSY1186 with Banl, a new plasmid, pSY1262, was generated. This plasmid contains a unique Banl site which allows for the direct ligation of fragments containing Banl ends obtained by polymerization of monomers. This plasmid has been used to generate plasmids containing inserts for the following proteins: SELP1, 2, 3, and Slp4.

Production and Purification of SlpIII

Cell Culture

E. coli are cultured in the following medium:

|  | g/l |
| --- | --- |
| yeast extract | 20 |
| casamino acids | 20 |
| peptone | 20 |
| gelatin peptone | 20 |
| $KH_2PO_4$ | 2 |
| $K_2HPO_4$ | 2 |
| $Na_2HPO_4 \cdot 7H_2O$ | 2 |
| glucose | 2 |
| ampicillin | 0.1 |

An overnight culture (500 ml - 1 L) which had been grown at 30°C was used to inoculate 375 L of media contained in a 500 L fermentor. Fermentor conditions include a tachometer reading of 100 rpm, vessel back pressure of 5 psi and an air flow of 170 l/min in order to maintain dissolved $O_2$ at greater than 50%.

Glucose (1 gm/l) and ampicillin (0.05 g/l) were added to the fermentation when the culture reached an $OD_{650}$ of 1.0 and again at 2.0. When the culture reached an $OD_{650}$ of 2.0 the temperature was increased to 42°C for 10 min and then lowered to 38°C for 2 hours. The culture was then chilled to 10°C and cells were harvested by centrifugation in a continuous centrifuge and frozen at -70°C until processed. Yields from two separate fermentations were 7.3 kg and 5.2 kg wet weight of cells.

It should be noted that other media can be used and, with different plasmids, various selection conditions can be imposed (i.e., substitution of kanamycin selection for ampicillin). These conditions have been used in laboratory scale fermentations (10 L volumes).

Cell Lysis

Method 1. Cells are thawed and suspended to a concentration of 1 kg wet weight/6 1 in 50 mM Tris-HCl pH 7.0, 1 mM EDTA and broken by 2 passages through an APR Gaulin cell disrupter at 8000 psi. During this lysis procedure the cells are kept cold with an ice bath. The cell lysate is then centrifuged at 26,000xg with a continuous centrifuge, such as the T2-28 rotor in a Sorvall RC5B refrigerated centrifuge operated at 4°C. Under these conditions greater than 90% of the SlpIII produced can be found in the pellet. The supernate does contain some product which can be recovered by $NH_4SO_4$ precipitation as described below. The pellet is extracted with LiBr as described below.

Method 2. Frozen cells are thawed and resuspended to a concentration of 1 kg wet weight/6 I in 50 mM Tris-HCl pH 7.0, 10 mM EDTA, and 5 mM PMSF to inhibit protease activity. Cells are stirred in this buffer at room temperature for 0.5 to 2 hours, then lysozyme is added to a concentration of 1 g/l and incubation is continued for 20 min. β-Mercaptoethanol is then added to 70 mM and the detergent NP40 is then added to a final concentration of 1% for 20 min while continuously stirring the cell suspension. Then $MgCl_2$ is added to 50 mM followed by DNAse at a concentration of 1 mg/l and incubation is continued at room temperature for 20 min. The cell lysate is then centrifuged as in method 1 at 26,000xg in a continuous centrifuge and the supernatant is collected and passed through the continuous centrifuge a second time at 26,000xg. The supernate resulting from this second centrifugation contains <5% of the total SlpIII, but what is there can be recovered with $NH_4SO_4$ as described below. The pellets resulting from the 1st and 2nd 26,000xg centrifugations are combined and extracted with LiBr as described below.

Method 3. For this method, a strain of E. coli is used that contains a second plasmid which encodes the T7 phage lysozyme. This plasmid is compatible with the plasmid encoding the S1pIII gene and the drug resistance determinant. The strain is grown in the same medium and under the same conditions as in the first two methods. However, due to the production of the T7 lysozyme inside the cells, their cell wall is weakened and they can be easily lysed at the completion of the fermentation by the addition of EDTA to >100 mM and NP40 to a concentration of from 0.5 to 1.0% v/v. Lysis can also be achieved by the addition of chloroform (20 ml per liter) of fermentation broth instead of NP40. Alternatively, cells may be collected by centrifugation prior to lysis, resuspended to 1 kg wet weight/6 1 in Tris-EDTA as described in the first two methods and then lysed by the addition of NP40 or chloroform. Following cell lysis by either method the lysate is centrifuged in a continuous rotor at 26,000xg as described in the first two methods. As with those methods, LiBr extraction of the pellet and $NH_4SO_4$ precipitation of the supernatant are used to recover the product.

Purification of SlpIII

The pellet obtained by centrifugation of the cell lysate at 26,000xg as described above is extracted with an equal volume of 9M LiBr. The salt solution is added and the pellet is evenly suspended by stirring at room temperature (RT). The mixture is stirred for 1 hour at RT after an even suspension is obtained. The mixture is then centrifuged at 26,000xg in a continuous rotor at 4°C or at RT to generate a pellet and a supernatant fraction. The supernate is saved and the pellet is re-extracted with another equal volume of 9M LiBr as above. After mixing for 1 hour the mixture is centrifuged at 26,000xg and the supernate from this centrifugation is combined with the supernate from the first LiBr extraction and allowed to stand at 4°C overnight. Approximately 90% of the SlpIII contained in the cell lysate 26,000xg pellet is extracted by LiBr using this procedure.

After the LiBr extract stands overnight at 4°C a precipitate forms, is removed by centrifugation at 26,000xg and is discarded. The supernate is then placed in dialysis bags and dialyzed against several changes of $dH_2O$ for 2 days. As the LiBr is removed by dialysis the SlpIII product precipitates in the dialysis bags. The precipitate is collected by centrifugation and washed 2-3 times with $dH_2O$. The final washed product is centrifuged and dried by lyophilization.

For the recovery of SlpIII from the 26,000 g supernatant fractions, $NH_4SO_4$ precipitation is used. Solid $NH_4SO_4$ is slowly added to the sample which is maintained at 4°C, until 38% saturation is achieved (231 g/l). The mixture is then stirred at 4°C for 2-3 hours. The precipitate is recovered by centrifugation in a continuous flow centrifuge and washed 4-5 times with an equal volume of distilled $H_2O$ or with 0.5% SDS in $H_2O$. After each wash the precipitate is recovered by continuous centrifugation. The pellet becomes increasingly white with successive washes as contaminating protein is removed. SlpIII is recovered as a washed pellet and can be dried by lyophilization.

Trypsin Treatment Step of SlpIII

SlpIII was suspended in 50 mM Tris HCl, pH 8.0, 0.1 M NaCl buffer, and was placed in a 37°C water bath, and TPCK treated trypsin solution was mixed into the suspension. The final trypsin concentration was 0.1%. After 3 hours, the solution was centrifuged at 16,000xg for 15 min., the pellet was washed with a half equal volume of 0.5% SDS in $H_2O$ first, then with distilled water. After each wash the pellet was recovered by centrifugation. The final product was resuspended in water and kept at 4°C for further analysis.

With the trypsin treatment, SlpIII was purified to 99.4% purity.

Physical Measurements of SlpIII

Physical measurements of the purified silk-like proteins have been compared with those of Bombyx mori silk in order to establish that the repetitive amino acid polymers produced microbiologically accurately mimic the properties of naturally occurring polymers. Physical measurements were performed to confirm the model of anti-parallel chain pleated sheet conformation for the crystalline regions of Bombyx mori silk fibroin (Marsh, Corey and Pauling, Biochem. Biophys. Acta (1955) 16; Pauling and Corey, Proc. Natl. Acad. Sci. USA (1953) 39:247. Preliminary analysis of x-ray difraction patterns obtained from Slp films are consistent with those described by Fraser, MacRai, and Steward (1966) (Table 4). Circular Dichroic (CD) and Fourier transform infrared (FTIR) spectroscopic analysis of SlpIII are consistent with a high degree of extended β and β-turn conformations. Comparisons of the spectra obtained from SlpIII with that of naturally occurring silk fibroin in various solvents (Isuka and Young, Proc. Natl. Acad. Sci. USA (1966) 55:1175) indicate that SlpIII in solution consists of a mixture of the random and highly ordered structures seen in silk fibroins.

Table 4

| Material | a (A) | b (A) | c (A) |
|---|---|---|---|
| (AG)$_n$ | 9.42 | 6.95 | 8.87 |
| (AGAGSG)$_n$ | 9.39 | 6.85 | 9.05 |
| CTP fraction | 9.38 | 6.87 | 9.13 |
| Native fibroin | 9.40 | 6.97 | 9.20 |
|  | 9.44 | 6.95 | 9.30 |
| S1pIII | 9.38 | 6.94 | 8.97 |

Referenced in Fraser et al., J. Mol. Biol. (1966) 19:580.

Example 4

EBSI Gene Construction:

Six oligonucleotide strands were synthesized and purified as described previously.

        (HIII)    BanII          StuI

i.   5'AGCTGGGCTCTGGAGTAGGCCTG3'

ii.   5'AATTCAGGCCTACTCCAGAGCCC3'
   (ER1)    StuI          BanII

   (HIII)    BanI

iii.  5'AGCTTGGTGCCAGGTGTAGGAGTTCCGGGTGTAGGCGTTCCGGGAGTTGG
      TGTACCTGGAGTGGGTGTTCCAGGCGTAGGTGTGC3'

   (XmaI)

iv.  5'CCGGGCACACCTACGCCTGGAACACCCACTCCAGGTACACCAACTCCCGGA
      ACGCCTACACCCGGAACTCCTACACCTGGCACCA3'
                                      BanI

```
     (XmaI)                                    AhaII
v.  5'CCGGGGTAGGAGTACCAGGGGTAGGCGTCCCTGGAGCGGGTGCTGGTAG
    CGGCGCAGGCGCGGGCTCCGGAGTAGGGGTGCCG3'
         BanII                      BanI
```

```
         (ERI)   BanI           BanII
vi.  5'AATTCGGCACCCCTACTCCGGAGCCCGCGCCTGCGCCGCTACCAGCACCCG
     CTCCAGGGACGCCTACCCCTGGTACTCCTACC3'
            AhaII
```

Oligonucleotide strands (iii), (iv), (v) and (vi) were annealed and ligated with the DNA of plasmid pBSm13(+) (Stratagene) which had been digested with HindIII and EcoRI. The products of this ligation reaction were transformed into E. coli strain JM109. Transformant colonies were selected for resistance to ampicillin. Colonies were screened for their hybridization with $^{32}$P-labelled oligonucleotides (iii), (v). Plasmid DNA from several positively hybridizing clones was purified and sequenced. Two of the plasmids, pSY1292 and pSY1293, contained the sequence shown for oligonucleotides (iii), (v) and (iv), (vi). These sequences contained all of the nucleotides present in this synthetic oligonucleotides except one. A G:C basepair was missing at position 7 (iii). The lack of ths basepair obstructed one of the BanI sites. In order to introduce a second BanII site at the 5' end of the gene fragment, oligonucleotides (i) and (ii) were annealed and ligated with plasmid pBSm13(+) which had been digested with HindIII and EcoRI. Plasmid DNA from the transformant colonies resistant to ampicillin was purified. Two plasmids, pSY1295 and pSY1296, which were digestible with StuI, a unique site contained in the oligonucleotide sequence, were sequenced. They were both shown to contain the sequence shown for oligonucleotides (i) and (ii). Plasmid DNA from pSY1292 was digested sequentially with HindIII, SI nuclease, and EcoRI. The digestion products were separated by electrophoresis in an agarose gel and the DNA fragment of approximately 150 basepairs was excised from the gel. This DNA fragment was digested with plasmid DNA pSY1296 which had been digested with StuI and EcoRI. The products of this ligation reaction were transformed into E. coli strain JM109 and were selected for resistance to ampicillin. Colonies were screened for hybridization to $^{32}$P-labelled oligonucleotide (v). The plasmid DNA from two positively hybridizing clones was purified and sequenced. These plasmids were named PSY1297 and pSY1298. They contained the following sequence:

```
(HindIII) BanII
AGCTGGGCTCTGGAGTAGGTGTGCCAGGTGTAGGAGTTCCGGGTGTAGGCGTTCCGGGAG    60
TCGACCCGAGACCTCATCCACACGGTCCACATCCTCAAGGCCCACATCCGCAAGGCCCTC
```

```
                                                   XmaI
TTGGTGTACCTGGAGTGGGTGTTCCAGGCGTAGGTGTGCCCGGGGGTAGGAGTACCAGGGG    120
AACCACATGGACCTCACCCACAAGGTCCGCATCCACACGGGCCCCATCCTCATGGTCCCC
```

```
                                          BanII
TAGGCGTCCCTGGAGCGGGTGCTGGTAGCGGCGCAGGCGCGGGCTCCGGAGTAGGGGTGC    180
ATCCGCAGGGACCTCGCCCACGACCATCGCCGCGTCCGCGCCCGAGGCCTCATCCCCACG
```

```
    EcoRI
CGAATTC
GCTTAAG
```

EBSI Multimer Gene Assembly:

The BanI acceptor plasmid pSY937 was modified in order to accept BanII terminal cohesive DNA fragments. Two oligonucleotides were synthesized for this purpose.

```
           (BamHI)     DraI  SspI        NruI                       BanII
    vii. 5'GATCCTATGTTTAAATATTCTCGCGAACGTTTTTGTATGGGCTCGATGTGT .

         TACCGTGCGCATGGATATCAGCTG3'
              FspI      EcoRV    PvuII
```

```
           (BamHI) PvuII EcoRV      FspI                        BanII
    viii 5'GATCCAGCTGATATCCATGCGCACGGTAACACATCGAGCCCATACAAAAA

       .  CGTTCGCGAGAATATTTAAACATAG3'
              NruI          SspI  DraI
```

Oligonucleotides (vii) and (viii) were annealed and ligated with plasmid DNA pSY937 which was digested with BamHI. The products of this ligation were transformed into E. coli strain JM109 and colonies were selected for resistance to chloramphenicol. Transformant colonies were screened by hybridization to $^{32}$P-labelled oligonucleotide (vii). Plasmid DNA from two positively hybridizing clones, pSY1299 and pSY1300, contained the sequence shown for oligonucleotides (vii) and (viii), as determined by DNA sequencing.

Plasmid DNA pSY1298 was digested with BanII and the digestion fragments separated by agarose gel electrophoresis. The EBSI gene fragment, approximately 150 base pairs, was excised and purified by electroelution and ethanol precipitation. Approximately 1 μg of purified fragment was self-ligated in order to produce multimers ranging in size from 450 bp to 6,000 bp. The products of the self-ligation were then ligated with plasmid DNA pSY1299 which had been digested with BanII. The products of this ligation reaction were transformed into E. coli strain HB101. Transformants were selected for resistance to chloramphenicol. Plasmid DNA from individual transformants was purified and analyzed for increased size due to EBSI multimer DNA insertions. Ten clones (pSY1240-1249) with inserts ranging in size from 1.5 Kbp to 4.4 Kbp were obtained.

Expression of EBSI Multimer Gene:

One of these clones, pSY1248, which contained a 4 Kb EBSI multimer gene was recloned in the $\lambda P_R$ expression vector, pSY751. Plasmid DNA from pSY1248 was digested with NruI and PvuII, separated by agarose gel electrophoresis, and the DNA band corresponding to the EBSI multimer gene was excised and purified by NACS purification. DNA from plasmid pSY751 was digested with PvuII and ligated with the NruI-PvuII fragment from pSY1248. The products of this ligation were transformed into E. coli HB101, and the transformants selected for resistance to ampicillin. Two clones were isolated containing the new plasmid pSY1280. E. coli cells containing pSY1280 were grown at 30°C to an $OD_{600}$ of 0.7 and then shifted to 42°C for 1.5 hours. The proteins produced by these cells was analyzed by SDS-PAGE. The separated proteins were transferred to nitrocellulose paper and detected by immunoreactivity with anti-ELP rabbit serum. A strongly reactive protein band was observed with an apparent molecular weight of 120 kD.

The Ampicillin drug resistance gene of pSY1280 was substituted with the Kanamycin marker and the subsequent plasmid was called pSY1332. This plasmid was used in fermentation for the purification of EBSI. (See Methods)

```
    pSY1332/pSY1280      EBSI Protein      1413 AA    MW 113,159


    MDPVVLQRRDWENPGVTQLNRLAAHPPFASERFCMGS
    [(GVGVP)8 (GAGAGSGAGAGS)1]26
    MCYRAHGYQLSAGRYHYQLVWCQK
```

Purification of EBSI Protein:

E. coli strain HB101 containing plasmid pSY1280 was fermented in 10L volume. The cells were concentrated by filtration and further harvested by centrifugation. Pelleted cells were stored frozen at -70°C until processed. Frozen cells were thawed on ice and suspended in 4 ml of 50 mM Tris-HCl pH 7.0, 10 mM EDTA, 5 mM PMSF per gram wet weight of cells. The cells were broken by French pressing twice at 15,000 psi and then cooled to 0°C. The crude lysate was cleared by centrifugation at 26Kxg for 20 minutes. The supernatant proteins were precipitated by addition of solid ammonium sulfate to 20% of saturation (114 g/l). The precipitate was collected by centrifugation at 10Kxg for 10 minutes. The pellet was resuspended in 10 ml of $H_2O$ and dialyzed against 10 mM Tris pH 8.0, 0.15M NaCl at 4°C. The dialyzed solution was digested with 0.1% Trypsin (Sigma) for 1.5 hours at room temperature, and reprecipitated with 20% ammonium sulfate. The precipitated protein was resuspended in $H_2O$ and dialyzed against 10 mM Tris pH 7.0, 1 mM EDTA at 4°C. The protein purity of this sample was analyzed by amino acid compostion and determined to be 83%.

Elastic Properties of EBSI Protein:

The soluble preparation of semi-purified EBSI protein described above was incubated at 37°C for 30 minutes and centrifuged at 10Kxg for 10 minutes at room temperature. This treatment caused the EBSI protein to aggregate, become insoluble, and pellet into a translucent solid. The solid was resistant to mechanical disruption either by vortexing or by maceration using a glass rod. The solid could be cut with a razor blade into strips which exhibited a high degree of elasticity. They fully retained their shape after repeated extensions and relaxations. They resisted compression with no apparent irreversible deformation of structure.

EBSI Purification

EBSI sample (-70% pure) was dialyzed in 50 mM Tris HCl, 50 mM NaCl, pH 8.0 at 4°C overnight with one change of buffer. If precipitation was observed, the sample was centrifuged at 27,000xg for 15 min at 4°C. All remaining steps were performed at 4°C. The supernatant was applied to a DEAE-Sephacel column which had been equilibrated with 50 mM Tris HCl, 50 mM NaCl, pH 8.0. The flow through fractions which contained EBSI were collected and pooled. NaCl was added to the pooled fractions from DEAE-Sephacel column to make a final concentration of 2 M NaCl in the sample. Insoluble material was removed by centrifugation at 27,000xg for 20 min. The supernatant was then loaded onto Phenyl-Sepharose column which was equilibrated with 50 mM sodium phosphate buffer, pH 7.0, with 2 M NaCl. The column was washed extensively with buffer until no eluting protein was detected by $A_{280}$. The column was then eluted stepwise with 50 mM sodium phosphate buffer, pH 7.0 and finally with water. The EBSI active fractions were pooled and stored at 4°C for further analysis.
With the addition of these steps to the previous procedures, 100% pure EBSI was obtained.

Example 5

ELPI Construction and Expression

Two oligonucleotide strands were synthesized and purified as described in the Methods section.

```
        (EcoRI)  BanI                                                  SmaI
i)   5'-AATTCGGTGCCCGGTGTAGGAGTTCCGGGTGTAGGCGTTCCCGGGGTAG
     GCGTTCCGGGAGTAGGGGTGCCA-3'
                       BanI
```

```
        BanI                                               SmaI
ii)  3'-GCCACGGGCCACATCCTCAAGGCCCACATCCGCCAAGGGCCCCATCCGCA
     AGGCCCTCATCCCCACGGTTCGA-5'
            BanI (HindIII)
```

The two oligonucleotide strands were annealed and ligated with the DNA of plasmid pBS m13(+) (Stratagene) which had been digested with RENs HindIII and EcoRI.
The products of this ligation reaction were transformed into E. coli strain JM109. Transformant colonies were screened for their hybridization with $^{32}$P-labeled oligonucleotide (i). Plasmid DNA from positively hybridizing clones was purified and sequenced. One plasmid, pSY1287, contained the sequence shown for oligonucleotides (i) and (ii).

Plasmid DNA from pSY1287 was digested with BanI REN and the digestion fragments were separated by agarose gel electrophoresis. The ELPI gene fragment, approximately 60 bp, was excised and purified by NACS column. Approximately 1 µg of purified fragment was self-ligated in order to produce multimers ranging in size from 300 bp to 5000 bp.

The products of the self-ligation were then ligated with plasmid DNA pSY937 which had been digested with REN BanI. The product of this ligation reaction was transformed into E. coli strain HB101. Transformants were selected for resistance to chloramphenicol. Plasmid DNA from individual transformants was purified and analyzed for increased size due to ELPI multiple DNA insertions. Four clones (pSY1388-1391) with inserts ranging in size from 1.0 kbp to 2.5 kbp were obtained. These clones were recloned in the $\lambda P_R$ expression vector pSY751. The clones obtained (pSY1392-1395) were used for expression of ELPI.

The ELPI protein had the following amino acid composition:

```
pSY1395      ELPI Protein      859 AA      MW 72,555


MDPVVLQRRDWENPGVTQLNRLAAHPPFARNILAIRW
[(VPGVG)4]40  VPWTRVDLSAGRYHYQLVWCQK
```

SELP1 Gene Construction and Expression

Two oligonucleotide strands were synthesized and purified as described in the Methods section.

```
             FspI PvuII  SnaBI  (PstI)
     (i)    5'-GTGCGCAGCTGGTACGTAGCTGCA-3'


             (PstI)    PvuII
     (ii)   3'-ACGTC|ACGCGTCGACCATGCATCG-5'-
                    FspI         SnaBI
```

These oligonucleotide strands were annealed and ligated with plasmid pSY1304 which had been digested with PstI REN (pSY1304 differs from pSY857 by having a monomeric unit in place of the trimeric unit of pSY857). Plasmid DNA from transformant colonies resistant to chloramphenicol was purified. One plasmid, pSY1365, which was digestible with REN SnaBI, was sequenced and proven to be correct.

ELPI gene fragment purified a's described (ELPI construction and expression) was treated with Mung Bean Nuclease as described by supplier (Stratagene). The DNA fragments mixture was then ligated with plasmid DNA PSY1365 which had been digested sequentially with RENs FspI, SnaBI and calf intestinal phosphatase. The products of this ligation reaction were transformed into E. coli strain HB101 and were selected for resistance to chloramphenicol. Plasmid DNA from individual transformants was purified and analyzed for the ELPI monomer DNA insertion. Two plasmids, pSY1366 A and B, were sequenced. They were both shown to contain the ELPI DNA sequence in the correct orientation.

Plasmid DNA pSY1366 was digested with REN BanI and the DNA fragment containing the SELP1 monomer was gel purified. To create multimers, 1 µg of the SELP1 DNA fragment was self-ligated. Multimers were obtained ranging in size from 500 bp to 10 kbp. The SELP1 multimers were cloned into the BanI site of pSY1262. Positive clones were characterized by gel electrophoresis for the size of the inserted multimer and used for expression and protein analysis.

```
pSY1396    SELP1 Protein    2025 AA    MW 148,212
```

$$MDPVVLQRRDWENPGVTQLNRLAAHPPFASDPMGAGS \quad (GAGAGS)_6$$
$$[GAA(VPGVG)_4 \ VAAGY \ (GAGAGS)_9]_{24}$$
$$GAA(VPGVG)_4 \ VAAGY \ (GAGAGS)_2 \ GAGAMDPGRYQLSAGRYHYQLVWCQK$$

SELP2 - Monomer Construction

Plasmid DNA pSY1298 was digested with BanII REN and the EBSI gene fragment was purified as described previously. The EBSI monomer fragment was ligated into pSY1304 (pSY937 containing a monomer of SlpIII, constructed as pSY857) which had been digested with BanII REN and treated with calf intestinal phosphatase).

The products of the ligation mixture were transformed in E. coli strain HB101. Transformants were selected for resistance to chloramphenicol. After restriction analysis of several isolates, one plasmid was chosen, pSY1301, containing a DNA fragment corresponding to the EBSI monomer gene.

SELP2 - Multiple Gene Assembly and Expression

Plasmid DNA pSY1301 was digested with REN BanI and the DNA fragment containing the SELP2 "monomer" was gel purified. To create multimers, 1 µg of the SELP2 DNA fragment was self-ligated. Multimers were obtained greater than 12 kb in size.

The SELP2 multimers were cloned into the BanI site of pSY1262. Positive clones were characterized by gel electrophoresis for the size of the inserted multimer. The clones with inserts ranging in size from 1.5 kb to 11 kb were selected. Plasmid DNA pSY1372 containing an insert of 6 kb (18 repeats) was used for further analysis and protein purification.

SELP2 - Protein Purification

E. coli strain HB101 containing plasmid pSY1372 was fermented according to the procedure described in Methods for fermentation. The cells were harvested by centrifugation. Pelleted cells were stored frozen at -70°C until processed. Frozen cells were thawed on ice and suspended in 4 ml of 50 mM Tris-HCl, pH 7.0, 10 mM EDTA, 5 mM PMSF per gram wet weight of cells. The cells were broken by passing through a Gaulin cell disrupter at 8,000 psi. The crude lysate was cleared by centrifugation at 26,000xg for 20 min. The supernatant, which contained >75% of the SELP2 protein, was precipitated by addition of 20% ammonium sulfate (114 g/L). The precipitate was collected by centrifugation at 10,000xg for 10 min. The pellet was resuspended in 10 ml of $H_2O$ and dialyzed against 10 mM Tris pH 8.0, 0.15 M NaCl at 4°C. The dialyzed material was centrifuged at 26,000x for 15 min in order to collect the insoluble fraction of protein which contained approximately 10% of the SELP2 protein. This insoluble protein pellet was washed twice in 0.2% SDS at 50°C for 30 min with occasional shaking. The insoluble protein was collected each time by centrifugation at 26,000xg for 15 min. followed by a wash of 50% ethanol. The final protein pellet was resuspended in water and analyzed by Western blot analysis and amino acid composition. By Western blot the SELP2 protein appears to be homogeneous in size consistent with its large molecular weight (>150 kd). By amino acid composition the SELP2 preparation is approximately 80% pure and the observed molar ratio of amino acids (Ser:Gly:Ala:Pro:Val:Tyr) agrees very closely with the expected composition as predicted from the SELP2 sequence present in pSY1372.

```
pSY1372    SELP2 Protein    2055 AA    MW152,354
```

$$MDPVVLQRRDWENPGVTQLNRLAAHPPFASDPMGAGS \quad (GAGAGS)_2 \ (GVGVP)_8$$
$$[(GAGAGS)_6 \ GAAGY \ (GAGAGS)_5 \ (GVGVP)_8]_{17}$$
$$(GAGAGS)_6 \ GAAGY \ (GAGAGS)_2 \ GAGAMDPGRYQLSAGRYHYQLVWCQK$$

SELP3 - Construction and Expression

Plasmid DNA pSY1301 was partially digested with REN HaeII and the digestion fragments separated by agarose gel electrophoresis. The larger DNA fragments were excised and purified by NACS column. The purified fragments were self-ligated, the ligation reaction was heated at 70°C for 15' to inactivate the T4 DNA ligase and eventually digested with REN PstI. The digestion mixture was then transformed into E. coli strain JM109. Transformants were selected for resistance to chloramphenicol. Plasmid DNA from individual transformants was purified and analyzed for: (1) resistance to REN PstI; and (2) deletion of 60 bp HaeII fragment contained within the SELP2 gene fragment. One clone (pSY1377) satisfied both requirements. Plasmid DNA pSY1377 was digested with REN BanI and the DNA fragment containing the SELP3 monomer was gel purified. To create multimers, 1 µg of the SELP3 DNA fragment was self-ligated. Multimers were obtained ranging in size from 500 bp to 10 kbp. The SELP3 multimers were cloned into the BanI site of pSY1262. Positive clones were characterized by gel electrophoresis for the size of the inserted multimer and used for expression and protein analysis.

```
pSY1397    SELP3 Protein    2257 AA    MW 168,535
```

$$\text{MDPVVLQRRDWENPGVTQLNRLAAHPPFASDPMGAGS (GAGAGS)}_2$$
$$[(GVGVP)_8 (GAGAGS)_8]_{24}$$
$$(GVGVP)_8 (GAGAGS)_5 \text{ GAGAMDPGRYQLSAGRYHYQLVWCQK}$$

SLP4 - Construction and Expression

Plasmid DNA pSY1304 (pSY857 with a single monomeric unit as distinct from the trimeric unit of pSY857) was partially digested with REN HaeII and the digestion fragments separated by agarose gel electrophoresis. The larger DNA fragments were excised and purified by NACS column. The purified fragments were self-ligated, the ligation reaction was heated at 70°C for 15' to inactivate the T4 DNA ligase and eventually digested with REN PstI. The digestion mixture was then transformed into E. coli strain JM109. Transformants were selected for resistance to chloramphenicol. Plasmid DNA from individual transformants was purified and analyzed for: (1) resistance to REN PstI; and (2) deletion of 60 bp HaeII fragment contained within the SELP2 gene fragment. One clone (pSY1378) satisfied both requirements. Plasmid DNA pSY1378 was digested with REN BanI and the DNA fragment containing the SLP4 monomer was gel purified. To create multimers, 1 µg of SLP4 DNA was self-ligated. Multimers were obtained ranging in size from 300 bp to 6 kbp. The SLP4 multimers were cloned into the BanI site of pSY1262. Positive clones were characterized by gel electrophoresis for the size of the inserted multimer and used for expression and protein analysis.

```
pSY1398    SLP4 Protein    1101 AA    MW 76,231
```

$$\text{MDPVVLQRRDWENPGVTQLNRLAAHPPFASDPMGAGS }[(GAGAGS)_6]_{27}$$
$$(GAGAGS)_4 \text{ GAGAMDPGRYQLSAGRYHYQLVWCQK}$$

As is evident from the above results, highly repetitive sequences can be prepared, cloned, and used for expression to produce a wide variety of products which may mimic natural products, such as silk and other proteins and antigens. In addition, novel systems are provided for controlling the expression of the peptide under inducible conditions in a variety of hosts. In this manner, new proteinaceous products can be provided which provide for new properties or may closely mimic the properties of naturally occuring products.

**Bibliography**

1. Maniatis, T., Fritsch, E.F. and Sambrook, J. 1982. -Molecular Cloning: A Laboratory Manual. Cold Spring Harbor Laboratory, Cold Spring Harbor, NY.

2. Laemmli, U.K. 1970. Nature (London), 227:680-685.

3. Applied Biosystems User Bulletin. 1984. No. 13.

4. Matteucci, M.D. and Caruthers, M.H. 1981. Journal Amer. Chem. Soc., 103:3185-3319.

5. McBride, L.J. and Caruthers, M.H. 1933. Tetrahedron Letters, 24:245-248.

6. Smith, 1980. Methods in Enzymology, 65:371-379.

7. Vieira, J. and Messing, J. 1982. Gene, 19:259-268.

8. Anagnostopouls, C. and Spizizen, J. 1981. J. Bacteriol., 81741-746.

9. Davanloo, P., Rosenberg, A.H. Dunn, J.J. and Studier, F.W. 1984. Proc. Natl. Acad. Sci. USA, 81:2035-2039.

10. Rosenbluh, A., Banner, C.D.B., Losick, R. and Fitz-James, P.C. 1981. J. Bacteriol., 148:341-351.

11. Sadaie, Y., Burtis, K.C. and Doi, R. 1980. J. Bacteriol., 141:1178-1182.

12. Queen, C. 1983. J. Applied Molecular Genetics, 2:1-10.

13. Ferrari, F.A., Trach, K. and Hoch, J.A. 1985. J. Bacteriol., 161:556-562.

14. Johnson, W.C., Moran, C.P. and Losick, T.R. 1983. Nature (London), 302:800-804.

15. Studier, W.F. and Moffat, B.A. 1986. J. Mol. Biol., 189:113-130.

16. Goldfarb, D.S., Doi, R.H. and Rodriguez, R.L. 1981. Nature (London), 293:309-311.

17. Ferrari, F.A., Nguyen, A., Lang, D. and Hoch, J.A. 1983. J. Bacteriol., 154:1513-1515.

18. Lacey, R.W. and Chopra, I. 1974. J. Med. Microbiology, 7:285-297.

19. Norrander, J., Kempe, T. and Messing, J. 1983. Gene, 26:101-106

20. Sanger, F., Nicklen, S. and Coulson, A.R. 1977. Proc. Natl. Acad. Sci. USA, 74:5463-5467.

21. Biggin, M.D., Gibson, T.J. and Hong, G.F. 1983. Proc. Natl. Acad. Sci. USA, 80:3963-3965.

22. Zagursky, R.J., Baumeister, K., Lomax, N. and Berman, M.L. 1985. Gene Anal. Techn., 2:89-94.

23. Sanger, F. and Coulson, A.R. 1978. FEES Letters, 87:107-110.

24. Sadler, J.R., Techlenburg, M. and J. L. Betz. 1980. Plasmids containing many tandem copies of a synthetic lactose operator. Gene 8:279-300.

All publications and patent applications mentioned in this specification are indicative of the level of skill of those skilled in the art to which this invention pertains.
The invention now being fully described, it will be apparent to one of ordinary skill in the art that many changes and modifications can be made thereto within the scope of the appended claims.

## EP 0 293 443 B1

**Claims**

1. A method of synthesising a recombinant DNA sequence which encodes a protein of at least about 10 kDal, the protein comprising repeating units of from 4 to 30 amino acids, each repeating unit having at least three different amino acids and at least two of the same amino acid, the method comprising the stages of:

   (a) preparing a monomer DNA sequence comprising different nucleotide sequences which, relying on codon redundancy, respectively encode two or more of the same repeating units from a fibrous protein; and
   (b) multimerising the monomer DNA sequence to provide said recombinant DNA sequence.

2. A method according to claim 1, wherein the stage of preparing said monomer DNA sequence includes linking different DNA sequences.

3. A method according to claim 1 or claim 2 wherein the monomer DNA sequence is prepared in a cloning vector and is excised from the cloning vector by restriction enzyme digestion prior to multimerisation.

4. A method according to any preceding claim wherein the stage of preparing a monomer DNA sequence includes the steps of synthesising different double-stranded DNA sequences and cloning them into respective cloning vectors.

5. A method according to any preceding claim wherein the stage of preparing the monomer DNA sequence includes the step of sequentially linking further said nucleotide sequences to a 5' or 3' terminus of a prior said nucleotide sequence.

6. A method according to any preceding claim including the step of sequencing the monomer DNA sequence prior to multimerisation.

7. A method according to any preceding claim including the step of sequencing the different nucleotide sequences prior to the stage of preparing the monomer DNA sequence.

8. A method according to any preceding claim wherein the monomer has protruding termini which are complementary to each other.

9. A method according to any preceding claim wherein the encoded protein comprises a repeating unit of GVGVP or SGAGAG.

10. A method according to any preceding claim wherein the encoded protein is at least about 15 kDal.

11. A method according to any preceding claim wherein the repeating units are of from 4 to 25 amino acids.

12. A method according to any preceding claim wherein the repeating units are of from 4 to 8 amino acids.

13. A method according to any preceding claim wherein at least 25% of the repeating units are the same.

14. A method according to claim 13 wherein at least 40% of the repeating units are the same.

15. A method according to claim 14 wherein at least 60% of the repeating units are the same.

16. A method according to any preceding claim wherein no more than 95% of the repeating units are the same.

17. A method according to claim 16 wherein no more than 90% of the repeating units are the same.

18. A method according to any preceding claim wherein different monomers respectively encoding different amino acid repeat units are multimerised.

19. A method according to any one of claims 1 to 17 wherein a monomer encoding different amino acid repeat units is multimerised.

**20.** A method of preparing a protein of at least about 10 kDal comprising a repeating unit of from 4 to 30 amino acids from a fibrous protein, the method comprising the steps of introducing a recombinant DNA sequence prepared according to the method of any preceding claim into a host cell, and growing the host cell thereby to express the protein.

**21.** A method of preparing an expression vector encoding a protein of at least 10 kDal comprising repeat units of from 4 to 30 amino acids, the method comprising the step of:

inserting a DNA sequence prepared according to the method of any one of claims 1 to 19 into an expression vector functional for expression in an expression hosts.

**22.** A method of preparing an expression host capable of producing a protein of at least 10 kDal comprising repeat units of from 4 to 30 amino acids, the method comprising the step of:

introducing an expression vector prepared according to the method of claim 21 into a said expression host.

**23.** A method of producing a protein of at least 10 kDal comprising repeat units of from 4 to 30 amino acids, the method comprising the step of:

growing an expression host prepared according to the method of claim 22 thereby to express the protein.

**24.** A method according to claim 20 or 23 including the further step of isolating the protein thus expressed.

**25.** A method according to any one of claims 20 to 24 wherein the recombinant DNA sequence is introduced into a structural gene of the host cell or vector.


**Patentansprüche**

**1.** Verfahren zum Synthetisieren einer rekombinanten DNA-Sequenz, die für ein Protein mit zumindest etwa 10 kDal kodiert, wobei das Protein Grundeinheiten aus 4 bis 30 Aminosäuren umfasst, wobei jede Grundeinheit zumindest drei verschiedene Aminosäuren und zumindest zwei derselben Aminosäure aufweist, wobei das Verfahren folgende Schritte umfasst:

(a) das Herstellen einer monomeren DNA-Sequenz, die unterschiedliche Nucleotidsequenzen umfasst, die, basierend auf Codon-Redundanz, jeweils für zwei oder mehr derselben Grundeinheiten eines Faserproteins kodieren; und
(b) das Multimerisieren der monomeren DNA-Sequenz, um die rekombinante DNA-Sequenz bereitzustellen.

**2.** Verfahren nach Anspruch 1, worin der Schritt des Herstellens der monomeren DNA-Sequenz das Verbinden unterschiedlicher DNA-Sequenzen umfasst.

**3.** Verfahren nach Anspruch 1 oder 2, worin die monomere DNA-Sequenz in einem Klonierungsvektor hergestellt wird und vor der Multimerisation durch Restriktionsenzym-Verdau aus dem Klonierungsvektor ausgeschnitten wird.

**4.** Verfahren nach einem der vorangegangenen Ansprüche, worin der Schritt des Herstellens einer monomeren DNA-Sequenz die Schritte des Synthetisierens unterschiedlicher doppelstrangiger DNA-Sequenzen und deren Klonierung in jeweilige Klonierungsvektoren umfasst.

**5.** Verfahren nach einem der vorangegangenen Ansprüche, worin der Schritt des Herstellens der monomeren DNA-Sequenz den Schritt des aufeinander folgenden Bindens weiterer dieser Nucleotidsequenzen an einen 5'- oder 3'-Terminus einer der vorherigen Nucleotidsequenzen umfasst.

**6.** Verfahren nach einem der vorangegangenen Ansprüche, das den Schritt des Sequenzierens der monomeren DNA-Sequenz vor der Multimerisation umfasst.

**7.** Verfahren nach einem der vorangegangenen Ansprüche, das den Schritt des Sequenzierens der unterschiedlichen Nucleotidsequenzen vor dem Schritt der Herstellung der monomeren DNA-Sequenz umfasst.

8. Verfahren nach einem der vorangegangenen Ansprüche, worin das Monomer überstehende Termini aufweist, die zueinander komplementär sind.

9. Verfahren nach einem der vorangegangenen Ansprüche, worin das kodierte Protein eine Grundeinheit aus GVGVP oder SGAGAG umfasst.

10. Verfahren nach einem der vorangegangenen Ansprüche, worin das kodierte Protein etwa 15 kDal aufweist.

11. Verfahren nach einem der vorangegangenen Ansprüche, worin die Grundeinheiten 4 bis 25 Aminosäuren lang sind.

12. Verfahren nach einem der vorangegangenen Ansprüche, worin die Grundeinheiten 4 bis 8 Aminosäuren lang sind.

13. Verfahren nach einem der vorangegangenen Ansprüche, worin zumindest 25 % der Grundeinheiten dieselben sind.

14. Verfahren nach Anspruch 13, worin zumindest 40 % der Grundeinheiten dieselben sind.

15. Verfahren nach Anspruch 14, worin zumindest 60 % der Grundeinheiten dieselben sind.

16. Verfahren nach einem der vorangegangenen Ansprüche, worin nicht mehr als 95 % der Grundeinheiten dieselben sind.

17. Verfahren nach Anspruch 16, worin nicht mehr als 90 % der Grundeinheiten dieselben sind.

18. Verfahren nach einem der vorangegangenen Ansprüche, worin unterschiedliche Monomere, die jeweils für unterschiedliche Aminosäure-Grundeinheiten kodieren, multimerisiert werden.

19. Verfahren nach einem der Ansprüche 1 bis 17, worin ein Monomer, das für unterschiedliche Aminosäure-Grundeinheiten kodiert, multimerisiert wird.

20. Verfahren zur Herstellung eines Proteins mit zumindest etwa 10 kDal, das eine Grundeinheit eines Faserproteins aus 4 bis 30 Aminosäuren umfasst, wobei das Verfahren die Schritte des Einführens einer rekombinanten DNA-Sequenz, die nach einem Verfahren nach einem der vorangegangenen Ansprüche hergestellt wurde, in eine Wirtszelle und des Züchtens der Wirtszelle, um das Protein dadurch zu exprimieren, umfasst.

21. Verfahren zur Herstellung eines Expressionsvektors, der für ein Protein mit zumindest 10 kDal kodiert, das Grundeinheiten aus 4 bis 30 Aminosäuren umfasst, wobei das Verfahren folgenden Schritt umfasst:

   das Insertieren einer DNA-Sequenz, die nach einem Verfahren nach einem der Ansprüche 1 bis 19 hergestellt wurde, in einen Expressionsvektor, der für die Expression in einem Expressionswirt funktionell ist.

22. Verfahren zur Herstellung eines Expressionswirts, der zur Produktion eines Proteins mit zumindest 10 kDal fähig ist, das Grundeinheiten aus 4 bis 30 Aminosäuren umfasst, wobei das Verfahren folgenden Schritt umfasst:

   das Einführen eines nach einem Verfahren nach Anspruch 21 hergestellten Expressionsvektors in den Expressionswirt.

23. Verfahren zum Produzieren eines Proteins mit zumindest 10 kDal, das Grundeinheiten aus 4 bis 30 Aminosäuren umfasst, wobei das Verfahren folgenden Schritt umfasst:

   das Züchten eines nach dem Verfahren nach Anspruch 22 hergestellten Expressionswirts, um dadurch das Protein zu exprimieren.

24. Verfahren nach Anspruch 20 oder 23, das den weiteren Schritt des Isolierens des so exprimierten Proteins umfasst.

25. Verfahren nach einem der Ansprüche 20 bis 24, worin die rekombinante DNA-Sequenz in ein Strukturgen der Wirtszelle oder einen Vektor eingeführt wird.

35

**Revendications**

1. Méthode de synthèse d'une séquence d'ADN recombinant qui code pour une protéine d'au moins environ 10 kDal, la protéine comprenant des unités récurrentes de 4 à 30 acides aminés, chaque unité récurrente ayant au moins trois acides aminés différents et au moins deux du même acide aminé, la méthode comprenant les stades de :

   (a) préparer une séquence d'ADN monomère comprenant différentes séquences de nucléotides qui, en reposant sur la redondance des codons, codent respectivement pour deux ou plusieurs des mêmes unités récurrentes à partir d'une protéine fibreuse ; et

   (b) multimériser la séquence d'ADN monomère pour produire ladite séquence d'ADN recombinant.

2. Méthode selon la revendication 1, où le stade de préparation de ladite séquence d'ADN monomère comprend l'enchaînement de différentes séquences d'ADN.

3. Méthode selon la revendication 1 ou la revendication 2 où la séquence d'ADN monomère est préparée dans un vecteur clonant et est excisée du vecteur clonant par digestion par enzyme de restriction avant multimérisation.

4. Méthode selon toute revendication précédente où le stade de préparation d'une séquence d'ADN monomère comprend les étapes de synthétiser différentes séquences d'ADN bicaténaire et de les cloner en vecteurs clonants respectifs.

5. Méthode selon toute revendication précédente où le stade de préparer la séquence d'ADN monomère comprend l'étape d'enchaîner séquentiellement encore lesdites séquences de nucléotides à un terminal 5' ou 3' avant ladite séquence de nucléotides.

6. Méthode selon toute revendication précédente comprenant l'étape de séquencer la séquence d'ADN monomère avant multimérisation.

7. Méthode selon toute revendication précédente comprenant l'étape de séquencer les différentes séquences de nucléotides avant le stade de préparation de la séquence d'ADN monomère.

8. Méthode selon toute revendication précédente où le monomère a des extrémités en saillie qui sont complémentaires l'une de l'autre.

9. Méthode selon toute revendication précédente où la protéine codée comprend une unité récurrente de GVGVP ou SGAGAG.

10. Méthode selon toute revendication précédente où la protéine codée a au moins 15 kDal.

11. Méthode selon toute revendication précédente où les unités récurrentes ont de 4 à 25 acides aminés.

12. Méthode selon toute revendication précédente où les unités récurrentes ont de 4 à 8 acides aminés.

13. Méthode selon toute revendication précédente où au moins 25% des unités récurrentes sont les mêmes.

14. Méthode selon la revendication 13 où au moins 40% des unités récurrentes sont les mêmes.

15. Méthode selon la revendication 14 où au moins 60% des unités récurrentes sont les mêmes.

16. Méthode selon toute revendication précédente où pas plus de 95% des unités récurrentes sont les mêmes.

17. Méthode selon la revendication 16 où pas plus de 90% des unités récurrentes sont les mêmes.

18. Méthode toute revendication précédente où différents monomères codant respectivement pour différentes unités récurrentes d'acides aminés sont multimérisés.

19. Méthode selon l'une quelconque des revendications 1 à 17 où un monomère codant pour différentes unités récurrentes d'acides aminés est multimérisé.

**20.** Méthode de préparation d'une protéine d'au moins environ 10 kDal comprenant une unité récurrente de 4 à 30 acides aminés d'une protéine fibreuse, la méthode comprenant les étapes d'introduire une séquence d'ADN recombinant préparée selon la méthode de toute revendication précédente dans une cellule hôte et de faire croître la cellule hôte pour ainsi exprimer la protéine.

**21.** Méthode de préparation d'un vecteur d'expression codant pour une protéine d'au moins 10 kDal comprenant des unités récurrentes de 4 à 30 acides aminés, la méthode comprenant l'étape de :

insérer une séquence d'ADN préparée selon la méthode de l'une quelconque des revendications 1 à 19 dans un vecteur d'expression fonctionnel pour l'expression dans des hôtes d'expression.

**22.** Méthode de préparation d'un hôte d'expression capable de produire une protéine d'au moins 10 kDal comprenant des unités récurrentes de 4 à 30 acides aminés, la méthode comprenant l'étape de :

introduire un vecteur d'expression préparé selon la méthode de la revendication 21 dans ledit hôte d'expression.

**23.** Méthode de production d'une protéine d'au moins 10 kDal comprenant des unités récurrentes de 4 à 30 acides aminés, la méthode comprenant les étapes de :

faire croître un hôte d'expression préparé selon la méthode de la revendication 22 pour ainsi exprimer la protéine.

**24.** Méthode selon la revendication 20 ou 23 comprenant l'autre étape d'isoler la protéine ainsi exprimée.

**25.** Méthode selon l'une quelconque des revendications 20 à 24 où la séquence d'ADN recombinant est introduite dans un gêne de structure de la cellule hôte ou du vecteur.

# FIG. 1

```
                                                        ┌→β-lactamase
  m  t  m  i  t  p  s  l  g  c  r  s  t  l  e  d  p │h  f  r
ATGACCATGATTACGCCAAGCTTGGGCTGCAGGTCGACTCTAGAGGATCCCCATTTCCGT
                  :          :      :      :      :
               HindIII  PstI  SalI  XbaI  BamHI

                                        -1 +1 of mature β-lactamase
  v  a  l  i  p  f  f  a  a  f  c  l  p  v  p  a  h
GTCGCCCTTATTCCCTTTTTTGCGGCATTTTGCCTTCCTGTTTTTGCTCAC...
```

# FIG. 2A

Immunoblot with antibody to B-lactamase

kD    1   2   3   4   5   6   7

— trimer
— dimer

__ unprocessed
enzyme

— mature enzyme

43►
25►
18►

# FIG. 2B

Immunoblot with antibody to gly-ala peptide

kD    1   2   3   4   5   6   7

— trimer
— dimer

— processed
trimer
— mature enzyme

43►
25►
18►

# FIG. 3

T7gp10/silkI fusion: immunoblot with anti-silk Ab

## FIG. 4A

uninduced
induced
uninduced
induced
uninduced
induced

gp10/silkI –
fusion

— 43 kD

— 25 kD

— 18 kD

## FIG. 4C

T7gp9/silkI fusion:
Coomassie blue stain

## FIG. 4B

T7gp9/silkI fusion:
immunoblot with anti-silk Ab

uninduced
induced
uninduced
induced
uninduced
induced

kilodaltons
MW markers
uninduced
induced

gp9/silkI –
fusion

— 97

— 68

— 43

— 25

— 18

97 –

68 –

43 –

25 –

18

14 –

—gp9/silkI
fusion

41

## FIG. 5

pGR71-43
NdeI
CAT
SalI

pUC13
AMP
ORI
XbaI

8. XbaI digest
9. Fill in ends

10. NdeI digest
11. SalI digest
12. Purify 1 kb fragment
13. Fill in ends

14. Ligate

pSY630
HincII
CAT
AMP
ORI
SmaI

pSY605
EcoRI
NdeI
P spoVG
HindIII
AMP
ORI

pSY964
EcoRI
NdeI
T7 RNAP
NdeI
AMP
ORI

1. EcoRI digest
2. NdeI partial digest
3. Purify 3 kb fragment

4. EcoRI digest
5. NdeI digest
6. Purify 3 kb fragment

7. Ligate

pSY649
EcoRI
NdeI
T7 RNAP
NdeI
P spoVG
HindIII
AMP
ORI
PvuI

15. PvuI digest
16. Fill in ends

17. HincII digest
18. SmaI digest
19. Purify 1.2 kb frag.

20. Ligate

pSY856
NdeI
T7 RNAP
NdeI
P spoVG
HindIII
EcoRI
ORI
CAT

# FIG. 6

Timecourse for accumulation of the kanamycin-
resistance gene product with the T7 system.

# FIG. 7

(1) Partial AhaIII digestion
(2) Recircularization

(3) XhoII digestion
(4) Insertion of adapter sequence:

```
          d  p  m  f  k  y  s  r  d  p  m  g  a  m  d  p  g  r  y  q  l
GATCCTATGTTTAAATATTCTCGCGATCCGATGGGTGCCATGGACCCGGGTCGATATCAGCTG
      GATACAAATTTATAAGAGCGCTAGGCTACCCACGGTACCTGGGCCCAGCTATAGTCGACCTAG
      BamHI  AhaIII SspI   NruI      BanI  StyI   AvaI     EcoRV   BamHI
                                           NcoI   SmaI             PvuII
```

5. BanI digest
6. Purify 180 bp "monomer"
7. Multimerization

8. BanI digest

β-galactosidase/silkIII fusion: Amido black stain

FIG. 9A

MW markers   751--30°C   751--42°C   980--30°C   980--42°C   MW markers

— 200

— 97

— 68

— 43

B. β-galactosidase/silkIII fusion: immunoblot with anti-silk Ab

FIG. 9B

751--30°C   751--42°C   980--30°C   980--42°C

— 97

— 68

— 43

# FIG. 8

# FIG. IO

(BamHI)  SmaI (EcoRI)

1. Synthesize EBSI
2. Assemble monomer gene fragment

pBS M13
BamHI
EcoRI
Amp
ori

3. BamHI, EcoRI digest

4. Ligate

pSY1298
BanII
EBSI
BanII
Amp
ori

pSY1299
BanII
ori
CAT

1. BanII digest
2. Purify EBSI gene monomer fragment
3. Self ligate

4. BanII digest

5. Ligate

pSY1248
NruI
ori
CAT
EBSI
PvuII

1. NruI, PvuII digest
2. Purify EBSI multimers

pSY751
PvuII
β-gal
Amp
ori
CAT
PvuII
PvuII

3. PvuII digest

4. Ligate

pSY1280
EBSI
Amp
ori
CAT

47